# FASCICULE DE BREVET EUROPEEN

(11) **EP 0 946 741 B1**
(45) Date de publication et mention de la délivrance du brevet: **15.02.2006**
(21) Numéro de dépôt: 97947083.8
(22) Date de dépôt: 18.11.1997
(51) Int. Cl.: C12N 15/86, C12N 7/01

(54) **PROCEDE DE PRODUCTION DE VIRUS RECOMBINANTS**
VERFAHREN ZUR HERSTELLUNG VON REKOMBINANTEN VIREN
METHOD FOR PRODUCING RECOMBINANT VIRUS

(30) Priorité: 22.11.1996 FR 9614278
(43) Date de publication de la demande: 06.10.1999
(73) Titulaire: CENTELION, 94400 Vitry Sur Seine (FR)
(72) Inventeur: LEBLOIS-PREHAUD, Hélène, F-78280 Guyancourt (FR); PERRICAUDET, Michel, F-28320 Ecrosnes (FR); VIGNE, Emmanuelle, F-94200 Ivry sur Seine (FR); YEH, Patrice, F-75005 Paris (FR)
(74) Mandataire: Bouvet, Philippe
(86) Numéro de dépôt international: PCT/FR1997/002073
(87) Numéro de publication internationale: WO 1998/022607

(56) Documents cités:
- WO-A-96/07734
- WO-A-96/09074
- T. C. PEAKMAN ET AL: "HIGHLY EFFICIENT GENERATION OF RECOMBINANT BACULOVIRUSES BY ENZYMATICALLY MEDIATED SITE-SPECIFIC IN VITRO RECOMBINATION" NUCLEIC ACIDS RESEARCH, vol. 20, no. 3, 11 février 1992, pages 495-500, XP000670349
- L. KARAYAN ET AL: "OLIGOMERIZATION OF RECOMBINANT PENTON BASE OF ADENOVIRUS TYPE 2 AND ITS ASSEMBLY WITH FIBER IN BACULOVIRUS-INFECTED CELLS" VIROLOGY, vol. 202, no. 2, 1994, pages 782-795, XP002012045

## Description

La présente invention concerne une méthode pour la production de virus recombinants. Elle concerne également des constructions utilisées pour la mise en oeuvre de cette méthode, les cellules productrices, et les virus ainsi produits. Ces virus sont utilisables comme vecteur de clonage et/ou d'expression de gènes in vitro, ex vivo ou in vivo.

Les vecteurs d'origine virale sont largement utilisés pour le clonage, le transfert et l'expression de gènes in vitro (pour la production de protéines recombinantes, la réalisation de tests de criblage, l'étude de la régulation de gènes, etc), ex vivo ou in vivo (pour la création de modèles animaux, ou dans des approches thérapeutiques). Parmi ces virus, on peut citer notamment les adénovirus, les virus adéno-associés (AAV), les rétrovirus, les virus de l'herpès ou encore de la vaccine.

La famille des Adénoviridae est largement répandue chez les mammifères et les oiseaux et regroupe plus de cent sérotypes différents de virus non-enveloppés à ADN bicaténaire, possédant une capside de symétrie icosaédrique (Horwitz. In: Fields BN, Knipe DM, Howley PM, ed. Virology. Third édition ed. Philadelphia: Raven Publishers, 1996: 2149-2171). En plus de son innocuité l'adénovirus possède un tropisme cellulaire très large. Contrairement au rétrovirus dont le cycle est dépendant de la division cellulaire, il peut infecter les cellules en division active comme les cellules quiescentes et son génome se maintient sous forme épisomaie. De plus il peut être produit avec des titres élevés (10¹¹ ufp/ml). Ces atouts majeurs en on fait un vecteur de choix pour le clonage et l'expression de gènes hétérologues. Les adénovirus du groupe C, notamment de type 2 et 5, ainsi que les adénovirus canins de type CAV-2, dont la biologie moléculaire est la mieux connue, sont à l'origine des vecteurs actuellement utilisés.

L'adénovirus possède un génome linéaire de 36kb, terminé à chacune de ces extrémités par des séquences inversées répétées (ITR) de 103bp comprenant une origine de réplication ainsi qu'un signal d'encapsidation situé près de l'ITR gauche, (Shenk, Adenoviridae: The Viruses and Their Replication. In: Fields BN, Knipe DM, Howley PM, ed. Virology. Philadelphia: Raven publishers, 1996: 2111-2148). Trois familles de gènes s'expriment au cours du cycle viral:
- Les gènes précoces immédiats (E1, E2, E3 et E4) qui sont impliqués dans la régulation de gènes cellulaires permettant notamment l'entrée de la cellule en phase S (E1A) et l'inhibition de l'apoptose (E1B). Ils interviennent aussi dans la régulation de gènes viraux précoces ou tardifs au niveau de la transcription, de l'épissage ou du transport des ARN messagers (E1A, E2A, E4). Ils jouent aussi un rôle dans la réplication et dans l'échappement à la réponse immunitaire.
- Les gènes précoces retardés (plX et IVa2) sont liés à la régulation de la transcription des génes tardifs (IVa2) ou à l'assemblage du virion (pIX).
- Les gènes tardifs (L1 à L5) sont transcrits à partir du promoteur fort (MLP). Un transcrit primaire de 28kb permet de générer les transcrits correspondant aux différentes protéines structurales (core, penton, hexon) et non-structurales participant à l'assemblage et à la maturation des particules virales, par épissage alternatif et utilisation de 5 sites de polyadénylation.

Des vecteurs adénoviraux ont été utilisés pour le clonage et l'expression de gènes in vitro (Gluzman et al., Cold Spring Harbor, New York 11724, p. 187), pour la création d'animaux transgéniques (WO95/22616) pour le transfert de gènes dans des cellules ex vivo (WO95/14785; WO95/06120) ou encore pour le transfert de gènes dans des cellules in vivo (voir notamment WO93/19191, WO94/24297, WO94/08026).

Concernant les virus adéno-associés (AAV), il s'agit de virus à ADN de taille relativement réduite, qui s'intègrent dans le génome des cellules qu'ils infectent, de manière stable et relativement site-spécifique. Ils sont capables d'infecter un large spectre de cellules, sans induire d'effet sur la croissance, la morphologie ou la différenciation cellulaires. Par ailleurs, ils ne semblent pas impliqués dans des pathologies chez l'homme. Le génome des AAV a été cloné, séquencé et caractérisé. Il comprend environ 4700 bases, et contient à chaque extrémité une région répétée inversée (ITR) de 145 bases environ, servant d'origine de réplication pour le virus. Le reste du génome est divisé en 2 régions essentielles portant les fonctions d'encapsidation : la partie gauche du génome, qui contient le gène rep impliqué dans la réplication virale et l'expression des gènes viraux; la partie droite du génome, qui contient le gène cap codant pour les protéines de capside du virus.

L'utilisation de vecteurs dérivés des AAV pour le transfert de gènes in vitro et in vivo a été décrite dans la littérature (voir notamment WO 91/18088; WO 93/09239; US 4,797,368, US5;139,941, EP 488 528).

Concernant les rétrovirus, il s'agit de virus intégratifs, infectant sélectivement les cellules en division. Ils constituent donc des vecteurs d'intérêt pour des applications cancer ou resténose par exemple. Le génome des rétrovirus comprend essentiellement deux LTR, une séquence d'encapsidation et trois régions codantes (gag, pol et env). La construction de vecteurs recombinants et leur utilisation in vitro ou in vivo a été largement décrite dans la littérature : voir notamment Breakfield et al., New Biologist 3 (1991) 203; EP 453242, EP 178220, Bernstein et al. Genet. Eng. 7 (1985) 235; McCormick, BioTechnology 3 (1985) 689, etc.

Pour leur utilisation comme vecteurs recombinants, différentes constructions dérivées des virus ont été préparées, incorporant différents gènes d'intérêt. Dans chacune de ces constructions, le génome viral a été modifié de manière à rendre le virus incapable de réplication autonome dans la cellule infectée. Ainsi, les constructions décrites dans l'art antérieur sont des virus défectifs pour certaines régions de leur génome essentielles à la réplication. En particulier, s'agissant d'adénovirus, les constructions de première génération présentent une délétion dans/de la région E1, essentielle à la réplication virale, au niveau de laquelle sont insérées les séquences d'ADN hétérologue (Levrero et al., Gene 101 (1991) 195 ; Gosh-Choudhury et al., Gene 50 (1986) 161). Par ailleurs, pour améliorer les propriétés du vecteur, il a été proposé de créer d'autres délétions ou modifications dans le génome de l'adénovirus. Ainsi, une mutation ponctuelle thermosensible a été introduite dans le mutant ts125, permettant d'inactiver la protéine de 72kDa de liaison à l'ADN (DBP) codée par la région E2 (Van der Vliet et al., 1975). D'autres vecteurs comprennent une deletion d'une autre région essentielle à la réplication et/ou à la propagation virale, la région E4. Des vecteurs adénoviraux dans lesquels les régions E1 et E4 sont délétées possèdent un bruit de fond de transcription et une expression de gènes viraux très réduits. De tels vecteurs ont été décrits pas exemple dans les demandes WO94/28152, WO95/02697, WO96/22378). Par ailleurs, des vecteurs portant une modification au niveau du gène IVa2 ont également été décrits (WO96/10088). En outre, des vecteurs dits "adénovirus minimum" ou "pseudo-adénovirus" (ou encore AdΔ) ne comportant que les régions nécessaires en cis à la production du virus (séquences ITRs et d'encapsidation) et dépourvus de toute séquence virale codante ont également été décrits (WO94/12649, WO94/28152, WO95/02697), bien que leur production reste très difficile, comme expliqué ci-après.

S'agissant d'AAV, les vecteurs décrits sont généralement dépourvus de l'ensemble des régions codantes Rep et Cap, qui sont substituées par des acides nucléiques d'intérêt.

Dans les vecteurs recombinants dérivés des rétrovirus, les gènes gag, pol et env sont généralement délétés, en tout ou en partie, et remplacés par une séquence d'acide nucléique hétérologue d'intérêt. Par ailleurs, les rétrovirus recombinants peuvent comporter des modifications au niveau des LTR pour supprimer l'activité transcriptionnelle, ainsi que des séquences d'encapsidation étendues, comportant une partie du gène gag (Bender et al., J. Virol. 61 (1987) 1639).

Compte tenu de leur caractère défectif pour la réplication, la production de ces différents virus recombinants implique la possibilité de transcomplémenter les fonctions délétées du génome. La transcomplémentation est précisément à l'origine de difficultés majeures pour la production de ces virus, et notamment l'apport des fonctions de transcomplémentation.

Deux approches ont été développées à cet égard. La première repose sur la construction de lignées transcomplémentantes, dites lignées d'encapsidation. La seconde repose sur l'utilisation d'adénovirus helper ou de plasmides helper.

Différentes lignées d'encapsidation de virus défectifs ont été construites. Ces lignées sont capables de produire les fonctions déficientes dans le vecteur viral. Généralement, ces lignées comportent, intégrée dans leur génome, la ou les régions délétées du génome viral (E1,E2 et/ou E4 par exemple pour l'adénovirus; gag, pol et/ou env pour le rétrovirus, rep et/ou cap pour l'AAV).

L'une des lignées connues pour la production d'adénovirus défectifs est par exemple la lignée 293 dans laquelle une partie du génome de l'adénovirus a été intégrée. Plus précisément, la lignée 293 est une lignée de cellules embryonnaires humaines de rein contenant l'extrémité gauche (environ 11-12 %) du génome de l'adénovirus sérotype 5 (Ad5), comprenant ITTR gauche, la région d'encapsidation, la région E1, incluant E1a et E1b, la région codant pour la protéine pIX et une partie de la région codant pour la protéine pIVa2. Cette lignée est capable de trans-complémenter des adénovirus recombinants défectifs pour la région E1, c'est-à-dire dépourvus de tout ou partie de la région E1, et de produire des stocks viraux ayant des titres élevés. Cette lignée est également capable de produire, à température permissive (32°C), des stocks de virus comportant en outre la mutation E2 thermosensible. D'autres lignées cellulaires capables de complémenter la région E1 ont été décrites, basées notamment sur des cellules de carcinome de poumon humain A549 (WO94/28152) ou sur des rétinoblastes humains (Hum. Gen. Ther. (1996) 215). Par ailleurs, des lignées capables de trans-complémenter plusieurs fonctions de l'adénovirus ont également été décrites. En particulier, on peut citer des lignées complémentant les régions E1 et E4 (Yeh et al., J. Virol. 70 (1996) 559; Cancer Gen. Ther. 2 (1995) 322 ; Krougliak et al., Hum. Gen. Ther. 6 (1995) 1575) et des lignées complémentant les régions E1 et E2 (WO94/281 52, WO95/02697, WO95/27071).

Différentes lignées ont également été décrites pour la production de rétrovirus défectifs, généralement capables d'exprimer les gènes gag, pol et env. De telles lignées sont par exemple la lignée PA317 (US4,861,719), la lignée PsiCRIP (WO90/02806), la lignée GP+envAm-12 (WO89/07150 la lignée BOSC (WO94/19478), etc. Pour construire des rétrovirus recombinants comportant un acide nucléique d'intérêt, un plasmide comportant notamment les LTR, la séquence d'encapsidation et ledit acide nucléique est construit, puis utilisé pour transfecter une lignée d'encapsidation telle que décrite ci-dessus, capable d'apporter en trans les fonctions rétrovirales déficientes dans le plasmide. Les rétrovirus recombinants produits sont ensuite purifiés par des techniques classiques.

L'utilisation de lignées peut cependant présenter certains inconvénients. Ainsi, il est difficile, couteux et contraignant au niveau industriel de construire et de valider de telles lignées. En effet, ces lignées doivent être stables et compatibles avec des utilisations industrielles. De plus, les lignées décrites permettent difficilement d'écarter la production de virus réplicatifs contaminants (RCA). Par ailleurs, ces lignées ne permettent pas aujourd'hui, de manière satisfaisante pour une utilisation industrielle, de transcomplémenter des génomes viraux très fortement défectifs, comme par exemple des adénovirus minimum tels que décrits ci-avant. En effet, l'adénovirus possède un génome organisé en différentes unités de transcription dont la régulation spatio-temporelle est très complexe. La transcomplémentation d'un adénovirus délété de toutes les séquences virales codantes en exprimant chaque unité de transcription séparément, de façon constitutive ou conditionnelle, à partir d'une lignée cellulaire n'a pu être réalisée de manière satisfaisante jusqu'à présent. Ainsi, seule une faible proportion du génome correspondant aux régions E1, E4, pIX, et aux trois protéines codées par E2 (pol, DBP et p-TP) a été exprimée de façon constitutive à partir de lignées cellulaires. Le reste du génome correspond à l'unité majeure de transcription tardive (MLTU) qui produit tous les messagers des protéines structurales et non-structurales à partir d'un transcrit primaire de 28kb et est activée après la réplication du génome. Or, pour générer des adénovirus minimum, la transcomplémentation de ces régions est indispensable. Ces lignées ne permettent pas non plus d'obtenir des titres très élevés de rétrovirus recombinants.

La seconde approche consiste à co-transfecter avec le génome viral défectif une construction (plasmide ou adénovirus) apportant les fonctions de complémentation. En particulier les AAV recombinants défectis sont généralement préparés par co-transfection, dans un lignée cellulaire infectée par un virus auxiliaire humain (par exemple un adénovirus), d'un plasmide contenant une séquence nucléique d'intérêt bordée de deux régions répétées inversées (ITR) d'AAV, et d'un plasmide portant les fonctions de complémentation (gènes rep et cap) d'AAV. Des variantes ont été décrites dans les demandes WO95/14771: WO95/13365: WO95/13392 ou WO95/06743. L'inconvénient d'utiliser un adénovirus helper réside principalement dans les risques de recombinaison accrus entre le vecteur adénoviral et l'adénovirus helper, et dans la difficulté de séparer le recombinant du helper lors de la production et de la purification des stocks viraux. L'inconvénient d'utiliser un plasmide helper, par exemple un plasmide Rep/cap réside dans les niveaux de transfection obtenus, qui ne permettent pas de produire des titres élevés de virus.

Il a été également décrit dans la demande WO96/09074 un vecteur baculovirus comportant les séquences ITRs (integrative Terminal Repeat) de l'AAV de sorte à permettre son intégration. Le baculovirus tel que décrit dans celle demande comprend éventuellement le gène rep de AAV afin de faciliter l'excision et l'insertion de l'ADN dans le génome des cellules cibles.

La présente demande décrit maintenant un nouveau système de production de virus qui permet de pallier à ces inconvénients. Le système de l'invention repose sur l'utilisation d'un baculovirus pour apporter les fonctions de complémentation.

Le système de production selon l'invention permet de manière particulièrement avantageuse de s'affranchir de l'emploi de lignées de complémentation établies, d'éviter les problèmes de RCA, et de transcomplémenter des génomes hautement défectifs. En outre le système de l'invention est applicable à toute cellule infectable par le virus désiré et par un baculovirus, et offre ainsi une grande souplesse d'utilisation.

Un premier objet de l'invention réside donc dans un procédé de production de virus recombinants défectifs selon lequel on introduit dans une population de cellule compétentes, le génome du virus recombinant défectif et un baculovirus comportant tout ou une partie des fonctions nécessaires à la transcomplémentation du génome recombinant défectif.

Le procédé de l'invention repose donc sur utilisation d'un baculovirus pour apporter les fonctions complémentantes. Différentes approches sont possibles. On peut tout d'abord utiliser des cellules compétentes n'exprimant aucune fonction de transcomplémentation du génome recombinant défectif. Dans ce cas, il est possible d'utiliser soit un baculovirus comportant l'ensemble des fonctions nécessaires à la transcomplémentation du génome recombinant défectif, soit plusieurs baculovirus portant chacun une ou plusieurs des fonctions nécessaires à la transcomplémentation du génome recombinant défectif. Il est également possible d'utiliser une population de cellules compétentes capables de transcomplémenter déjà une ou plusieurs fonctions du génome recombinant défectif (lignée d'encapsidation). Dans ce cas, le ou les baculovirus utilisés apporteront uniquement les fonctions nécessaires à la transcomplémentation du génome recombinant défectif qui ne sont pas déjà transcomplémentées par les cellules compétentes.

Comme indiqué ci-avant, les avantages du système de l'invention sont nombreux en terme d'industrialisation (pas besoin de lignées, pas de RCA, etc), et en termes d'applications (production de virus recombinants portant tout type de délétion, et notamment d'adénovirus recombinants hautement défectifs). En outre, dans la mesure où le baculovirus ne se réplique pas dans les cellules humaines, la préparation virale obtenue n'est pas contaminée par le baculovirus. De plus, le baculovirus étant très éloigné phylogénétiquement des adénovirus, il n'y a pas de risques de recombinaison ou transcomplémentation entre les deux. Ce système permet donc, de manière avantageuse, de produire des stocks concentrés de virus défectifs, dépourvus de RCA. Ce système est tout particulièrement avantageux pour la production d'adénovirus recombinants défectifs.

Les baculovirus sont des virus enveloppés à ADN bicaténaire circulaire spécifiques d'invertébrés. Leur prototype, AcNPV, a un génome de 133kb. Il est très utilisé comme vecteur d'expression de gènes eucaryotes en cellules d'insectes, à partir de deux promoteurs forts [polyédrine (Ph) et P10], (King et Possee, The baculovirus expression system. London: Chapman&Hall, 1992.). AcNPV est capable d'infecter certaines cellules de mammifères mais le génome n'est ni transcrit ni traduit. Récemment, Hofmann et coll. (PNAS 92 (1995) 10099) ont montré que in vitro des cellules hépatocytaires peuvent être transduites par un baculovirus recombiné purifié exprimant le gène LacZ. Aucune toxicité cellulaire n'a été rapportée, même avec une multiplicité d'infection de 1000, et l'efficacité de transfection décrite dans cet article est de 50% environ pour une MOI de 100.

La demanderesse a maintenant montré qu'il est possible d'infecter différents types cellulaires avec un baculovirus recombiné. En particulier, la demanderesse a montré qu'il était possible, avec un baculovirus recombiné, d'infecter des cellules d'origine humaine telle que des cellules embryonaires immortalisées. La demanderesse a également montré qu'il est possible d'obtenir une efficacité de transduction très élevée (> 80%). La demanderesse a également montré qu'il est possible d'introduire, dans un baculovirus, des fonctions de complémentation d'un adénovirus, et d'exprimer ces fonctions dans une population de cellules compétentes. La demanderesse a ainsi permis de montrer que le baculovirus constitue un vecteur inerte pouvant être utilisé avantageusement pour le transfert et l'expression de fonctions de complémentation de virus dans des cellules de mammifères, notamment humaines. D'autres avantages du système de l'invention sont notamment (i) la grande capacité de clonage permettant de complémenter un génome adénoviral entier et (ii) le développement avancé de la technologie du baculovirus.

Le baculovirus portant les fonctions de complémentation du virus est également désigné dans ce qui suit baculovirus helper. Il peut comporter différents fonctions de complémentation du virus.

Ainsi, le baculovirus helper peut comporter la région E1 de l'adénovirus. Un Baculo-E1 peut être utilisé pour la production d'adénovirus de première génération, c'est-à-dire d'adénovirus défectifs pour la région E1 (AdΔE1), quel que soit son statut E3 (i.e. défectif AdΔE1,ΔE3, ou non). La production d'adénovirus recombinants défectifs de première génération (défectifs pour la région E1, et éventuellement E3) constitue une première application particulièrement avantageuse du procédé de l'invention. Comme indiqué ci-avant, différentes lignées ont été décrites dans la littérature capables de transcomplémenter la fonction E1 (cellules 293, cellules A549, cellules 911, etc). Cependant, il existe des zones d'homologie entre la région portant les fonctions de transcomplémentation intégrée dans le génome de la lignée et l'ADN du virus recombinant que l'on souhaite produire. De ce fait, au cours de la production, différents événements de recombinaison peuvent se produire, générant des particules virales réplicatives, notamment des adénovirus de type E1+. Il peut s'agir d'un événement simple de recombinaison suivi d'une cassure du chromosome, ou d'une double recombinaison. Ces deux types de modification conduisent à réintégrer dans son locus initial au sein du génome adénoviral la région E1 contenue dans le génome cellulaire. Par ailleurs, compte tenu des titres élevés de vecteur recombinant produits par la lignée 293 (supérieur à 10¹²), la probabilité que ces événements de recombinaison aient lieu est élevée. De fait, il a été constaté que de nombreux lots de vecteurs adénoviraux recombinants défectifs de première génération étaient contaminés par des particules virales réplicatives, ce qui peut constituer un inconvénient important pour des utilisations pharmaceutiques. En effet, la présence de telles particules dans des compositions thérapeutiques induirait in vivo une propagation virale et une dissémination incontrolée, avec des risques de réaction inflammatoire, de recombinaison, etc. Les lots contaminés ne peuvent donc être utilisés en thérapie humaine.

La présente invention permet de remédier à ces inconvénients. En effet, selon un mode de mise en oeuvre du procédé de l'invention, le génome de l'adénovirus recombinant défectif pour la région E1 et éventuellement E3 est introduit dans les cellules compétentes, ces cellules sont infectées, de façon simultanée ou non par un baculovirus comportant la région E1, la région E1 d'adénovirus présente dans le baculovirus et le génome de l'adénovirus recombinant défectif ne comportant aucune zone d'homologie (recouvrement) susceptible de donner lieu à une recombinaison. Selon ce mode de mise en oeuvre, il est ainsi possible de produire rapidement, sans lignée établie, des stocks d'adénovirus recombinants de première génération dépourvus de RCA. Par ailleurs, comme indiqué ci-après, les stocks d'adénovirus recombinants ainsi générés, dépourvus de RCA, peuvent être utilisés comme matériel de départ pour une nouvelle production, par co-infection dans les cellules compétentes avec un baculovirus.

Le baculovirus helper peut également comporter la région E2 de l'adénovirus, en tout ou en partie, notamment la région E2a et/ou E2b. Un baculo-E2 peut être utilisé pour produire, dans des cellules compétentes, des adénovirus défectifs pour la région E2 (Ad-ΔE2), et éventuellement pour la région E3 (Ad-ΔE2,ΔE3). En outre, dans des cellules compétentes capables de complémenter la région E1 de l'adénovirus, le baculo-E2 peut permettre la production d'adénovirus recombinants défectifs pour les régions E1 et E2 (Ad-ΔE1,ΔE2) et éventuellement E3 (Ad-ΔE1,ΔE2,ΔE3). De même, dans des cellules compétentes capables de complémenter les régions E1 et E4 de l'adénovirus (par exemple dans les cellules IGRP2), le baculo-E2 peut permettre la production d'adénovirus recombinants défectifs pour les régions E1, E2 et E4 (Ad-ΔE1,ΔE2,ΔE4) et éventuellement E3 (Ad-ΔE1,ΔE2,ΔE3,ΔE4).

Le baculovirus helper peut encore comporter la région E4 (en tout ou en partie) de l'adénovirus. Un baculo-E4 peut être utilisé pour produire, dans des cellules compétentes, des adénovirus défectifs pour la région E4 (Ad-ΔE4), et éventuellement pour la région E3 (Ad-ΔE4,ΔE3). En outre, dans des cellules compétentes capables de complémenter la région E1 de l'adénovirus, le baculo-E4 peut permettre la production d'adénovirus recombinants défectifs pour les régions E1 et E4 (Ad-ΔE1,ΔE4) et éventuellement E3 (Ad-ΔE1,ΔE4,ΔE3).

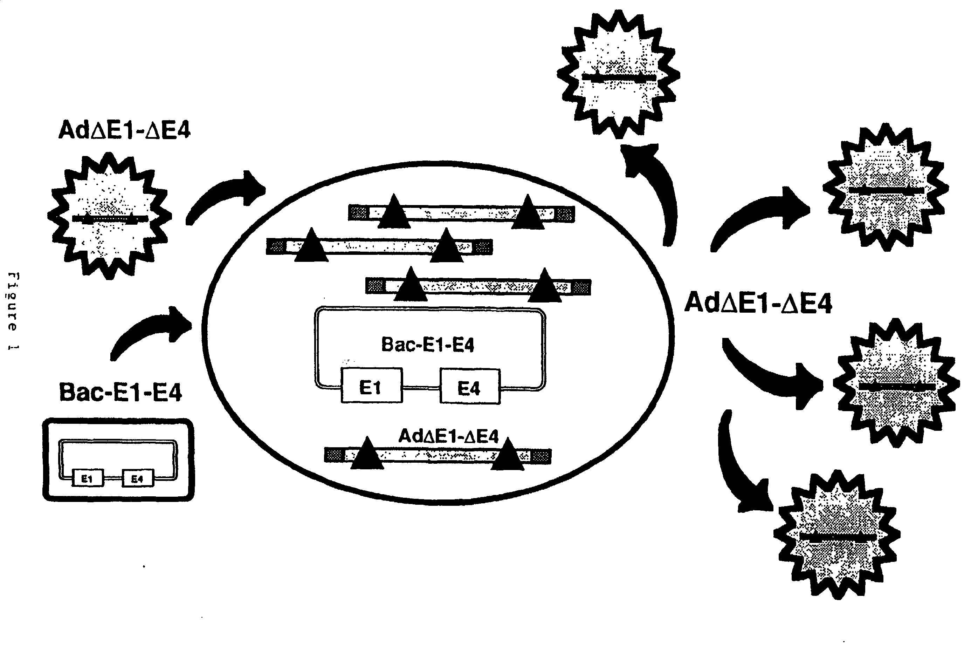

Le baculovirus helper peut aussi comporter les régions E1 et E4 (en tout ou en partie) de l'adénovirus. Un baculo-E1,E4 peut être utilisé pour produire, dans des cellules compétentes, des adénovirus défectifs pour les régions E1 et E4 (Ad-ΔE1,ΔE4) et éventuellement E3 (Ad-ΔE1,ΔE4,ΔE3), comme illustré sur la Figure 1.

En outre, pour générer des virus défectifs pour les régions E1 et E4, il est également possible d'utiliser deux baculovirus helper, l'un exprimant la fonction E1, l'autre la fonction E4, en tout ou en partie.

De la même manière, le baculovirus helper peut comporter les régions E1,E2 et E4 (en tout ou partie), et éventuellement les région portant les gènes tardifs (L1-L5).

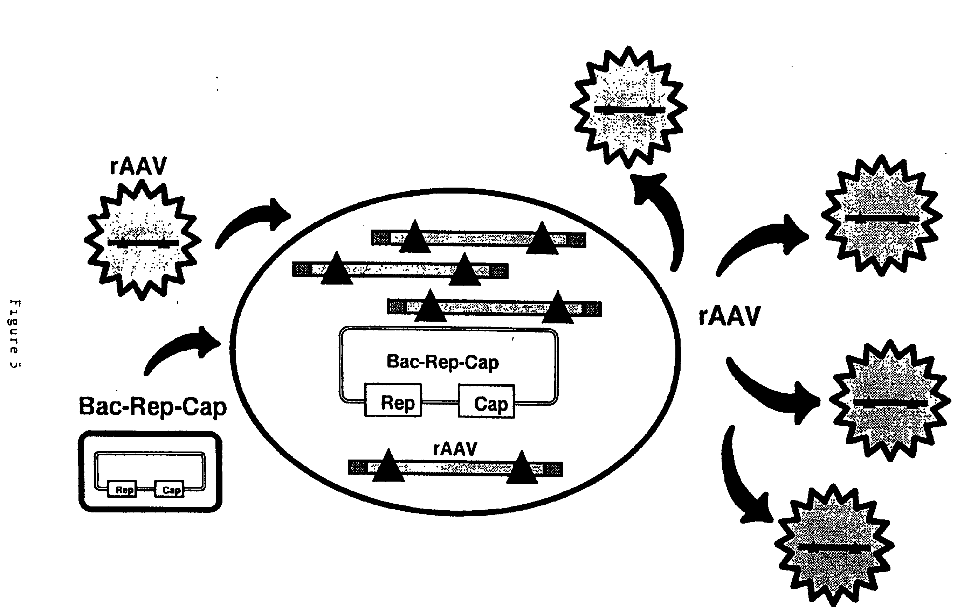

Le baculovirus helper peut également comporter les région Rep et/ou Cap de l'AAV. Un baculo-Rep/Cap permet ainsi de complémenter, dans une lignée de cellules compétentes, un génome d'AAV dépourvu de toute séquence virale codante (Figure 5).

Le baculovirus peut également comporter les région gag, pol et/ou env d'un rétrovirus. Un baculo-gag/pol/env permet ainsi de complémenter, dans une lignée de cellules compétentes, un génome rétroviral dépourvu de toute séquence virale codante. Il est également possible d'utiliser un baculovirus comportant les régions gag/pol et un deuxième baculovirus comportant la région env.

D'une manière générale, il est préférable que le génome du virus recombinant défectif et les régions de complémentation présentes dans le baculovirus ne présentent pas de recouvrement. Ceci permet en effet d'éviter les risques de recombinaison et ainsi, la génération de RCA. Ceci est particulièrement important pour la génération d'adénovirus de première génération (Ad-ΔE1). Dans ce cas, la région E1 introduite dans le baculovirus est définie de sorte qu'elle ne possède pas de séquence commune avec le génome recombinant. Pour ce faire, il est possible par exemple de déléter du génome recombinant une région plus grande que la région complémentante insérée dans le baculovirus, comme illustré dans les exemples. Ceci est également avantageux pour la génération d'adénovirus Ad-ΔE1,ΔE4.

Ainsi, dans un mode de réalisation particulier du procédé de l'invention, le génome du virus recombinant défectif est introduit dans les cellules compétentes, ces cellules sont infectées, de façon simultanée ou non, par un baculovirus comportant tout ou une partie des fonctions nécessaires à la complémentation du génome défectif, les fonctions de complémentation présentes dans le baculovirus et le génome du virus recombinant défectif ne comportant pas de zone d'homologie susceptible de donner lieu à une recombinaison. Avantageusement, le génome viral est un génome d'adénovirus recombinant défectif pour la région E1 et le baculovirus porte une région de l'adénovirus capable de trans-complémenter la région E1. Selon une autre variante, le génome viral est un génome d'adénovirus recombinant défectif pour les régions E1 et E4 et le baculovirus porte deux régions d'adénovirus capable de trans-complémenter lesdites régions ou deux baculovirus sont utilisés, l'un portant une région de l'adénovirus capable de trans-complémenter la région E1 et l'autre une région de l'adénovirus capable de trans-complémenter la région E4 sans zones d'homologies avec le génome adénoviral défectif.

Selon un mode de réalisation particulier, l'ensemble des régions codantes de l'adénovirus est porté par un ou plusieurs baculovirus helper. Selon un mode de réalisation plus particulier, on utilise un seul baculovirus helper comportant l'ensemble des régions codantes de l'adénovirus. Un tel baculovirus helper est ainsi utilisable pour transcomplémenter des adénovirus recombinants minimum. Un tel baculovirus peut notamment comporter l'ensemble d'un génome adénoviral, à l'exception de la région d'encapsidation et éventuellement des ITRs, comme illustré dans les exemples.

### Préparation des fonctions de complémentation

Les fonctions de complémentation introduites dans le baculovirus helper peuvent provenir de virus de sérotypes différents.

S'agissant d'adénovirus, il en existe en effet différents sérotypes dont la structure et les propriétés varient quelque peu, mais qui présentent une organisation génétique comparable. Plus particulièrement, les fonctions de complémentation utilisées pour la construction des baculovirus selon l'invention sont issues d'un adénovirus d'origine humaine ou animale.

Concernant les adénovirus d'origine humaine, on peut citer préférentiellement ceux classés dans le groupe C. Plus préférentiellement encore, parmi les différents sérotypes d'adénovirus humain, on préfère utiliser dans le cadre de la présente invention les adénovirus de type 2 ou 5 (Ad 2 ou Ad 5). Il est également possible d'utiliser des région provenant d'adénovirus de type 7 ou 12, appartenant aux groupes A et B. Parmi les différents adénovirus d'origine animale, on préfère utiliser dans le cadre de l'invention des adénovirus d'origine canine, et notamment toutes les souches des adénovirus CAV2 [souche manhattan ou A26/61 (ATCC VR-800) par exemple]. D'autres adénovirus d'origine animale sont cités notamment dans la demande WO94/26914 incorporée à la présente par référence.

Dans un mode préféré de mise en oeuvre de l'invention, la fonction de complémentation est issue d'un génome d'adénovirus humain du groupe C. De manière plus préférentielle, elle est issue du génome d'un adénovirus Ad2 ou Ad5.

Les régions portant les différentes fonctions de complémentation peuvent étre-obtenues, à partir d'un génome adénoviral, par coupures enzymatiques selon les méthodes connues de l'homme du métier. Ces régions peuvent éventuellement être modifiées pour réduire leur taille, ou remplacer certains éléments de régulation (promoteur, enhanceur, etc) par des éléments hétérologues. De manière générale, ces régions sont préparées comme suit. L'ADN d'un adénovirus est purifié par centrifugation sur gradient de chlorure de césium ou obtenu in vitro à partir d'un plasmide procaryote (WO96/25506) ou eucaryote (WO95/03400). L'ADN est alors clivé par des enzymes de restriction appropriés et les fragments obtenus, portant les fonctions de complémentation recherchées sont identifiés et sélectionnés. Le choix des enzymes de restriction utilisées dépend des fonctions de complémentation recherchées. Il est ensuite guidé par les cartes de restriction et les séquences publiées des génomes adénoviraux. Ainsi, la région E1 peut être isolée sous forme de fragments portant tous les cadres de lecture de E1A et E1B en aval du promoteur E1A. La région E4 peut être isolée sous forme de fragments portant l'ensemble des phases de lecture, ou seulement une partie d'entre-elles, et préférentiellement les phases ORF3 ou ORF6 ou ORF6-ORF6/7.

Des méthodologies similaires sont mises en oeuvre pour préparer les régions de complémentation d'AAV et de rétrovirus recombinants. Ainsi, les régions rep et/ou cap de l'AAV peuvent être obtenues par coupure enzymatique à partir de l'ADN viral isolé de différents sérotypes d'AAV. Il s'agit préférentiellement d'AAV-2. Pour les rétrovirus, les régions gag, pol et/ou env peuvent également être obtenues selon les techniques classiques de biologie moléculaire, à partir de différents types de rétrovirus tels que notamment le MoMuLV ("Murine Moloney Leukemia Virus"; encore désigné MoMLV), le MSV ("Murine Moloney Sarcoma Virus"), le HaSV ("Harvey Sarcoma Virus"); le SNV ("Spleen Necrosis Virus"); le RSV ("Rous Sarcoma Virus") ou encore le virus de Friend.

### Construction du baculovirus helper

Les fragments portant les régions de complémentation sont ensuite sous clonés dans un vecteur plasmidique permettant leur manipulation (digestions plus fines, PCR, additions de séquences de régulation, etc), par exemple dans un organisme procaryote ou eucaryote. Le fragment final obtenu, codant pour la ou les fonctions de complémentation, est alors introduit dans le baculovirus helper en utilisant les techniques classiques de biologie moléculaire. Précisément, le fragment est cloné entre deux séquences homologues à une région du génome d'un baculovirus, puis le fragment ou plasmide résultant est co-transfecté avec le génome d'un baculovirus dans des cellules d'insectes (classiquement Sf9 et Sf21, cellules de spodoptera frugiperda, mais également Tn-368 et High-Five™ BTI-TN-5B1-4 (Gibco), cellules de trichopulsia ni, ou toute autre cellule d'insecte permissive aux baculovirus et utilisable pour leur production). La recombinaison homologue entre le plasmide ou fragment et le génome du baculovirus génère le baculovirus recombinant désiré, qui peut être récupéré et purifié selon les méthodes classiques (voir notamment King et Possee: the baculovirus expression system. London: Chapman & Hall, 1992). Pour la construction des baculovirus recombinants, différents kits comportant des vecteurs navettes sont commercialisés et peuvent être utilisés en suivant les recommandations des fabricants. Notamment, on peut utiliser les vecteurs navettes pBAC commercialisés par la société Clontech, les vecteurs pAc (Verne et al., Bio/Technology 6 (1988) 47, Pharmingen, USA), les vecteurs pBlue-Bac (Invitrogen) ou encore les vecteurs pBSV (Boehringer). Les fonctions de complémentation peuvent ainsi être insérées en différents sites du baculovirus, et notamment dans le locus du gène de la polyhédrine ou du gène p10. Par ailleurs, différentes souches de baculovirus peuvent être utilisées, telles que notamment AcNPV ou Bombyx mori (Maeda et al., Nature 315 (1988) 592). D'autre part, le baculovirus utilisé peut être modifié pour améliorer/changer son tropisme. Il est en effet possible de moduler le tropisme des vecteurs viraux en modifiant leurs protéines de surface afin (i) de le restreindre par fusion des protéines virales avec un ligand spécifique (chaîne légère d'immunoglobuline, Gastrin-Releasing-Peptide) ou (ii) de l'élargir par formation de pseudotypes avec une glycoprotéine virale hétérologue [G du virus de la Stomatite Vésiculeuse (VSV)], [Liu et coll., J. Virol 70(4) (1996) 2497; Michael et coll., Gène Ther. 2 (1995) 660]. Récemment il a été montré que la glycoprotéine de surface du baculovirus (gp64) fusionnée à la gp120 du virus VIH était capable de se fixer sur le récepteur CD4 (Boublik et coll. Bio/Technology 13 (1995) 1079). Cette modification de la gp64 n'affecte pas la viabilité du baculovirus en cellules d'insectes. Une construction analogue avec la G de VSV devrait permettre d'améliorer le tropisme du baculovirus pour les cellules de mammifère et donc d'augmenter l'efficacité de transduction des cellules Huh7 ainsi que d'autres lignées cellulaires.

Dans le baculovirus helper, les fonctions de complémentation sont avantageusement placées sous le contôle d'un promoteur hétérologue (i.e. d'une origine différente du baculovirus), fonctionnel dans les cellules compétentes. Il apparait en effet que les promoteurs du baculovirus ne permettent pas d'obtenir des niveaux d'expression suffisants des fonctions de complémentation dans les cellules autres que les cellules d'insectes, et ne sont donc pas les plus appropriés pour les applications de l'invention. Le promoteur peut être tout d'abord le propre promoteur (promoteur homologue) des fonctions de complémentation du virus (promoteur E1A, E4, E2, MLP pour l'adénovirus, promoteurs P5 ou P19 de l'AAV, le promoteur du LTR du RSV, etc). Il peut également s'agir de tout promoteur d'origine différente fonctionnel dans la cellule compétente utilisée. A cet effet, on peut citer par exemple les promoteurs de gènes exprimés dans cette cellule, ou des promoteurs ubiquitaires connus comme par exemple le promoteur du gène PGK, le promoteur du immédiat précoce du CMV, le promoteur du gène TK du virus de l'herpès ou encore le promoteur du LTR du RSV. Il peut également s'agir de promoteur régulés, comme par exemple le promoteur du virus MMTV, un promoteur répondant aux hormones, par exemple de type GRE5, ou un promoteur régulé par la tétracycline (WO). Avantageusement, il s'agit d'un promoteur homologue, ubiquitaire fort ou inductible.

Ainsi, un autre objet de la présente invention concerne un baculovirus recombinant comprenant, inséré dans son génome, un acide nucléique codant pour une fonction de complémentation d'un virus placée sous le contrôle d'un promoteur hétérologue. Plus particulièrement, la fonction de complémentation est une protéine nécessaire à la production dudit virus, et dont la région codante est inactive (mutée, délétée, etc) dans le génome viral défectif. Pour les adénovirus, la fonction de complémentation est plus particulièrement choisie parmi tout ou partie des fonctions codées par les régions E1, E2, E4, L1-L5, pIX et IVa2 de l'adénovirus, seules ou en combinaisons. Pour l'AAV, il s'agit des fonctions codées par les régions Rep et/ou Cap; et pour le rétrovirus, gag, pol et/ou env. Avantageusement, l'acide nucléique correspond à une région d'un génome viral comportant la région codant pour la fonction de complémentation choisie. En particulier, il s'agit d'un fragment d'un génome d'adénovirus de sérotype Ad2 ou Ad5, MoMLV ou AAV-2. De manière particulièrement préférée, l'acide nucléique comprend également la région promotrice naturellement responsable de l'expression des fonctions de complémentation choisies.

A titre d'exemple particulier, la présente invention concerne un baculovirus comportant tout ou partie de la région E1 d'un adénovirus. Plus particulièrement, il s'agit d'un baculovirus comportant la région E1a, E1b ou E1a et E1b. La région E1 de l'adénovirus est localisée au niveau des nucléotides 104 (promoteur E1a) à 4070 (polyA E1b) de l'Ad5. En particulier, la boite TATA du promoteur E1a est localisée au niveau du nucléotide 470, le codon ATG de E1a au niveau du nucléotide 560, et le codon stop E1b au niveau du nucléotide 3511. On peut citer à titre d'exemple précis un baculovirus comportant un fragment 391-3511. Ce baculovirus helper est particulièrement adapté à la production d'adénovirus recombinants défectifs pour la région E1, portant une délétion plus large que ce fragment 391-3511. Notamment, il est adapté à la production d'adénovirus de première génération, sans RCA, portant une délétion dans la région E1 couvrant les nucléotides 383-3512 inclus.

Un autre exemple particulier de baculovirus selon l'invention comporte par exemple tout ou partie des régions E1 et E4 de l'adénovirus. La région E4 de l'adénovirus est constituée de 7 phases ouvertes de lecture, désignées ORF1, ORF2, ORF3. ORF4, ORF3/4, ORF6 et ORF6/7. Parmi les protéines codées par ces différentes ORFs, celles produites par ORF3 et ORF6 semblent permettre la "réplication" du virus, et donc la transcomplémentation d'un adénovirus défectif pour la région E4. même dans son intégralité. De ce fait, le baculovirus helper de l'invention comporte avantageusement toute la région E4 ou une partie seulement de celle-ci comportant au moins la phase ORF3 ou ORF6. Les différentes parties de la région E4 peuvent être obtenues par coupures enzymatiques ou modifiées selon les méthodes connues de l'homme du métier. En particulier, la phase de lecture ORF6 peut être isolée de la région E4 sous forme d'un fragment BglII-PvuII, correspondant aux nucléotides 34115-33126, et les phases de lecture ORF6-ORF6/7 peuvent être isolées de la région E4 sous forme d'un fragment BglII-BglII correspondant aux nucléotides 34115-32490 du génome de l'Ad5. Le baculovirus peut également comporter l'ensemble des phases de lecture ORF1-ORF7 (par exemple sous forme d'un fragment 32800-35826 ou 32811-35614 ou 32811-35640). Il est entendu que d'autres fragments peuvent être déterminés sur la base des séquences publiées des génomes adénoviraux. L'utilisation d'un baculovirus portant une unité réduite de la région E4 est avantageuse car permet la transcomplémentation d'un génome adénoviral défectif portant une délétion plus large de la région E4, donc sans zone d'homologie, et ainsi d'éviter toute possibilité de recombinaison.

Selon un premier mode de réalisation, l'acide nucléique codant pour la ou les fonctions de complémentation est introduit dans le baculovirus helper sous forme de cassette d'expression. Ce mode de réalisation est le plus simple à mettre en oeuvre. Il est particulièrement adapté à la production d'adénovirus recombinants défectifs pour des gènes précoces immédiats et pour la production de rétrovirus et d'AAV recombinants défectifs.

Selon un autre mode de réalisation, l'acide nucléique codant pour la ou les fonctions de complémentation est introduit dans le baculovirus helper sous forme d'une cassette excisable, générant une molécule réplicative dans la cellule compétente. La réplication de la cassette dans la cellule permet avantageusement d'augmenter le nombre de copies des gènes complémentant, et ainsi d'améliorer les niveaux de production du système. Ce mode de réalisation est particulièrement adapté à la production d'adénovirus recombinants très hautement défectifs, notamment défectifs pour les gènes de structure. En particulier, ce mode de mise en oeuvre est particulièrement adapté à la production d'adénovirus "minimum". En effet, la quantité de protéine structurale est un facteur limitant pour la production à titres importants d'adénovirus hautement défectifs (type adénovirus minimum). Ce mode de réalisation permet pour la première fois d'augmenter considérablement les niveaux intracellulaires de protéines transcomplémentantes, notamment de protéines structurales de l'adénovirus (codées par les régions L1 à L5), jusqu'à des niveaux compatibles avec la transcomplémentation d'adénovirus minimum.

Ainsi, la demanderesse a montré qu'il est possible de construire des baculovirus recombinants comprenant une région hétérologue susceptible d'être excisée dans une cellule, préférentiellement de manière inductible et régulée, pour générer une molécule circulaire et réplicative (de type épisomal).

L'excision est avantageusement réalisée par un mécanisme de recombinaison site-spécifique, et la réplication dans la cellule est assurée par une origine de réplication, indépendante de l'état de division cellulaire.

Plus préférentiellement, la recombinaison site-spécifique utilisée selon le procédé de l'invention est obtenue au moyen de deux séquences spécifiques qui sont capables de recombiner entre elles en présence d'une protéine spécifique, généralement désignée recombinase. Ces séquences spécifiques, disposées dans l'orientation appropriée, encadrent dans le baculovirus les séquences codant pour les fonctions de complémentation. Ainsi, l'invention a également pour objet un baculovirus recombinant comprenant, inséré dans son génome, au moins une région d'ADN encadrée par deux séquences permettant une recombinaison site-spécifique et positionnées en orientation directe, ladite région d'ADN comportant au moins une origine de réplication fonctionnelle dans les cellules compétentes et un acide nucléique codant pour une fonction de complémentation d'un virus.

Les séquences permettant la recombinaison utilisées dans le cadre de l'invention comprennent généralement de 5 à 100 paires de bases, et, plus préférentiellement, moins de 50 paires de bases. Elles peuvent appartenir à différentes classes structurales, et notamment à la famille de la recombinase du bactériophage P1 ou de la résolvase d'un transposon.

Parmi les recombinases appartenant à la famille de l'intégrase du bactériophage 1, on peut citer notamment l'intégrase des phages lambda (Landy et al., Science 197 (1977) 1147), P22 et F80 (Leong et al., J. Biol. Chem. 260 (1985) 4468), HP1 de Haemophilus influenzae (Hauser et al., J. Biol. Chem. 267 (1992) 6859), l'intégrase Cre du phage P1, l'intégrase du plasmide pSAM2 (EP 350 341) ou encore la FLP recombinase du plasmide 2 µm de la levure Saccharomyces cerevisiae.

Parmi les recombinases appartenant à la famille du transposon Tn3, on peut citer notamment la résolvase du transposon Tn3 ou des transposons gd, Tn21 et Tn522 (Stark et al., 1992) ; l'invertase Gin du bactériophage mu ou encore la résolvase de plasmides, telle que celle du fragment par de RP4 (Abert et al., Mol. Microbiol. 12 (1994) 131).

Selon un mode de réalisation préféré, dans les baculovirus recombinants de la présente invention, les séquences permettant la recombinaison site-spécifique sont dérivées d'un bactériophage. Plus préférentiellement, il s'agit des séquences d'attachement (séquences attP et attB) d'un bactériophage ou de séquences dérivées. Ces séquences sont capables de recombiner spécifiquement entre-elles en présence d'une recombinase désignée intégrase. A titre d'exemples précis, on peut citer notamment les séquences d'attachement des phages lambda, P22, F80, P1, HP1 de Haemophilus influenzae ou encore du plasmide pSAM2, ou 2 µm.

Encore plus préférentiellement, les séquences permettant la recombinaison site-spécifique sont représentées par le système de recombinaison du phage P1. Le phage P1 possède une recombinase du nom de Cre qui reconnaît spécifiquement une séquence nucléotidique de 34 paires de bases appelée site lox P (Stemberg et al., J. Mol. Biol. 150 (1981) 467). Cette séquence est composée de deux séquences palindromiques de 13pb séparées par une séquence conservée de 8pb. La recombinaison site-spécifique est avantageusement obtenue en utilisant des séquences LoxP ou des séquences dérivées, et la recombinanse Cre.

Le terme séquence dérivée inclut les séquences obtenues par modification(s) des séquences de recombinaison ci-dessus, conservant la capacité de recombiner spécifiquement en présence de la recombinase appropriée. Ainsi, il peut s'agir de fragments réduits de ces séquences ou au contraire étendus par addition d'autres séquences (sites de restriction, etc). Il peut également s'agir de variants obtenus par mutation(s), notamment par mutation(s) ponctuelle(s).

Selon un mode privilégié de l'invention, les séquences permettant une recombinaison site-spécifique sont donc des séquences LoxP du bactériophage P1, et la recombinaison est obtenue en présence de la protéine Cre. A cet égard, la recombinaison peut être obtenue par mise en contact direct des cellules compétentes avec la recombinase Cre, ou par expression du gène codant pour la recombinase Cre dans les cellules compétentes. Avantageusement, la recombinase Cre est produite dans la cellule par induction de l'expression du gène correspondant. Ainsi, le gène codant pour la recombinase est avantageusement placé sous contrôle d'un promoteur inductible, ou construit sous forme régulable. A cet égard, on utilise avantageusement une fusion entre Cre et le domaine de fixation d'hormones stéroïdiennes (oestradiol, progestérone, etc) permettant de réguler l'activité de Cre et donc d'induire l'événement de recombinaison (Metzger et al., PNAS 92 (1995) 6991). Plus généralement, l'expression de la recombinase peut être controlée par tout promoteur fort, régulé ou non. La cassette d'expression peut être transfectée dans les cellules compétentes, ou intégrée dans le génome des cellules compétentes, comme illustré dans les exemples.

Ce système permet donc de générer des molécules réplicatives produisant, dans les cellules compétentes, des niveaux élevés de fonction de complémentation de virus, notamment d'adénovirus. Ce type de construction est particulièrement adapté à la complémentation de génomes hautement défectifs, et en particulier de génomes adénoviraux défectifs pour les gènes tardifs. Ainsi, une construction particulière selon l'invention est représentée par un baculovirus comprenant, inséré dans son génome, au moins une région d'ADN encadrée par deux séquences LoxP positionnées en orientation directe, ladite région d'ADN comportant au moins une origine de réplication fonctionnelle dans les cellules compétentes et un acide nucléique codant pour une fonction de complémentation d'un adénovirus. Avantageusement, les fonctions de complémentation comprennent tout ou partie des gènes précoces immédiats présents dans les régions E1, E2 et E4. Encore plus préférentiellement, les fonctions de complémentation comprennent tout ou partie des gènes précoces immédiats et des gènes précoces retardés. Préférentiellement, les fonctions de complémentation permettent la complémentation d'un adénovirus recombinant dépourvu de toute séquence virale codante. En particulier, les fonctions de complémentation sont constituées de l'ensemble du génome adénoviral, à l'exception des ITRs et de la région de packaging. Selon une variante particulière, les fonctions de complémentation sont constituées d'un génome adénoviral complet dépourvu toutefois de la région de packaging (Ad.Psi-). Ce génome comprend en particulier les ITRs qui servent à la réplication du génome dans les cellules compétentes, après excision.

Pour assurer la réplication de la molécule épisomale, celle-ci contient donc une origine de réplication fonctionnelle dans les cellules compétentes utilisées. Cette origine de réplication est préférentiellement constituée des propres séquences ITR de l'adénovirus, qui permettent une amplification importante de la molécule. Il peut également s'agir d'une autre origine de réplication permettant, de préférence, une amplification d'un facteur supérieur à 20 de l'ADN viral dans la cellule compétente. On peut citer à titre illustratif l'origine OriP/EBNA1 du virus EBV ou la région E2 du virus du papillome. Il est entendu que les séquences ITR de l'adénovirus constituent un mode de réalisation préféré.

Pour la mise en oeuvre du procédé de l'invention, le ou les baculovirus helper sont généralement utilisés à une Multiplicité d'Infection (MOI) permettant d'infecter une large population des cellules, sans nuire de manière significative à la viabilité cellulaire. Généralement, celle-ci est plus particulièrement comprise entre 10 et 1000. La MOI correspond au nombre de particules virales par cellule. La MOI peut aisément être ajustée par l'homme du métier en fonction des cellules compétentes utilisées, sur la base essentiellement de deux critères : l'efficacité d'infection et la toxicité éventuelle. Avantageusement, la MOI utilisée pour le baculovirus helper est comprise entre 20 et 500.

### Introduction du génome viral

Comme indiqué ci-avant, le procédé de l'invention comprend l'introduction, dans des cellules compétentes, du baculovirus helper et du génome viral recombinant. A cet égard, le génome de-l'adénovirus recombinant défectif peut être introduit de différentes façons dans la cellule compétente.

Il peut tout d'abord s'agir d'un adénovirus recombinant défectif purifié, avantageusement dépourvu de RCA. Dans ce cas, les cellules compétentes sont infectées par l'adénovirus recombinant défectif et par le baculovirus helper. L'infection par l'adénovirus recombinant permet d'introduire dans la cellule compétente le génome correspondant, qui est ensuite amplifié et encapsidé pour produire des stocks à titre élevé, dépourvus de RCA. Ce mode de mise en oeuvre est particulièrement intéressant pour générer des virus de première génération (Ad-ΔE1; Ad-ΔE1,ΔE3). En effet, ces virus sont difficiles à produire à titres élevés, sans contamination par des RCA. Selon le procédé de l'invention, il est maintenant possible, en partant d'un adénovirus recombinant défectif de première génération, par co-infection dans une cellule compétente avec un baculovirus comportant la région E1, d'obtenir des stocks concentrés, de qualité élevée. Ce mode de réalisation est également avantageux pour la production de virus défectifs dans deux ou trois régions essentielles de leur génome (E1, E2, E4 notamment). D'une manière générale, ce mode de mise en oeuvre est avantageux car l'efficacité d'infection par l'adénovirus est très élevée (supérieure à l'efficacité de transfection par de l'ADN), et permet donc de générer des stocks concentrés. Dans ce mode de réalisation, l'adéncvirus recombinant et le baculovirus recombinants sont utilisés à des multiplicités d'infection (MOI) permettant d'infecter une large population des cellules, sans nuire de manière significative à la viabilité cellulaire. La MOl utilisée pour le baculovirus est celle mentionnée ci-avant (entre 10 et 1000). Concernant l'adénovirus, elle est avantageusement comprise entre 1 et 1000, de préférence entre 1 et 500, encore plus préférentiellement entre 1 et 100. La MOI utilisée pour l'adénovirus est également adaptée en fonction du type cellulaire choisi. La fourchette de MOI peut être aisément déterminée par l'homme du métier en utilisant par exemple un adénovirus et un baculovirus comportant un gène marqueur distinct, afin de mesurer l'efficacité d'infection et une éventuelle compétition. Plus préférentiellement, la MOI de l'adénovirus est inférieure à 50, par exemple comprise entre 1 et 20.

Selon un autre mode de réalisation particulièrement avantageux, le génome de l'adénovirus recombinant défectif est introduit sous forme d'ADN. Dans ce cas, le génome est introduit par transfection, éventuellement en présence d'agent facilitant la transfection (lipides, phosphate de calcium, etc). Le génome recombinant ainsi introduit peut être préparé in vitro selon différentes techniques, et en particulier chez E.coli (WO96/25506) ou dans une levure (WO95/03400). Ce mode de mise en oeuvre est surtout utile pour générer un premier lot de virus recombinant défectif, dépourvu de RCA, qui peut ensuite à son tour être utilisé pour produire des stocks de titre élevé selon le mode de réalisation précédent.

Le génome de fadénovirus recombinant défectif peut aussi être introduit en utilisant un autre baculovirus recombinant. Selon ce mode de réalisation, le génome de l'adénovirus recombinant défectif est préparé in vitro, par exemple comme indiqué ci-avant, puis introduit dans un baculovirus, sous la forme d'une cassette excisable dans la cellule compétente. Selon ce mode de mise en oeuvre, les cellules compétentes sont mises en présence d'un baculovirus portant le génome de l'adénovirus recombinant défectif, et d'un ou plusieurs baculovirus helper (portant les fonctions de complémentation). Ce mode de réalisation est particulièrement avantageux pour la production d'adénovirus recombinants hautement défectifs. Grace à ce système, il est en effet possible d'introduire dans la population de cellules compétentes des quantités élevées à la fois de génome recombinant hautement défectif et des fonctions de complémentation correspondantes.

A cet égard, un procédé de l'invention comprend donc la co-infection des cellules compétentes avec un baculovirus portant le génome de l'adénovirus recombinant défectif, et un ou plusieurs baculovirus helper portant les fonctions de complémentation. Les MOI utilisées dans ce mode de réalisation sont également comprises entre 10 et 1000 pour chacun des baculovirus utilisés.

Deux types de constructions ont été réalisées dans l'art antérieur pour la production d'adénovirus minimum : (1) le transgène (β.galactosidase) cloné entre les ITRs, bordés par un site de restriction unique ou (2) les ITR droit et gauche clonés en orientation directe en 5' du transgène (Fisher et coll., Virology 217 (1996) 11; Kumar-Singh et coll; Hum. Mol. Genet. 5 (1996) 913). Des adénovirus minimum ont été produits dans les cellules 293 par transfection de l'ADN linéarisé (1) ou circulaire (2), les protéines virales nécessaires à la réplication et à l'encapsidation du mini-génome étant apportées en trans par un virus helper (AdΔE1). Les adénovirus minimum se comportent comme des particules défectives interférentes (DI) et sont progressivement amplifiés au cours des passages successifs. Le problème majeur posé par l'utilisation de cette méthodologie est la séparation des deux types de particules produites responsable de la contamination des stocks par le virus helper, et les titres très faibles des adénovirus minimum ainsi obtenus (inférieurs à 10⁸ufp/ml).

La présente demande permet pour la première fois de générer des adénovirus minimum en utilisant un baculovirus pour délivrer le mini-génome adénoviral et un baculovirus pour apporter l'ensemble des fonctions de transcomplémentation (génome complémentant).

Le génome adénoviral recombinant est avantageusement introduit dans le baculovirus, entre deux séquence permettant une recombinaison site-spécifique dans les cellules compétentes, comme décrit pour le baculovirus helper.

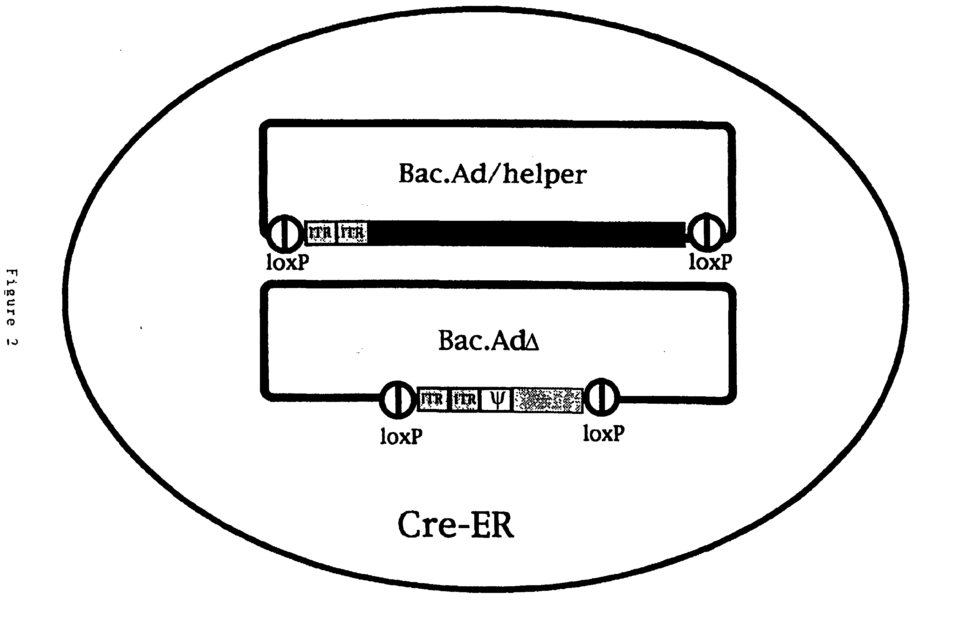

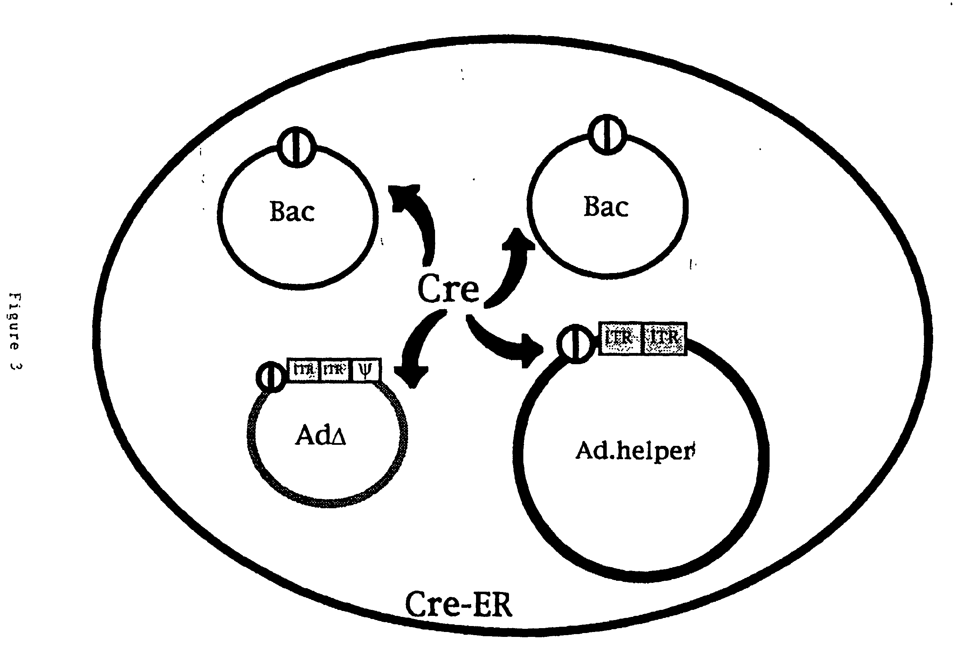

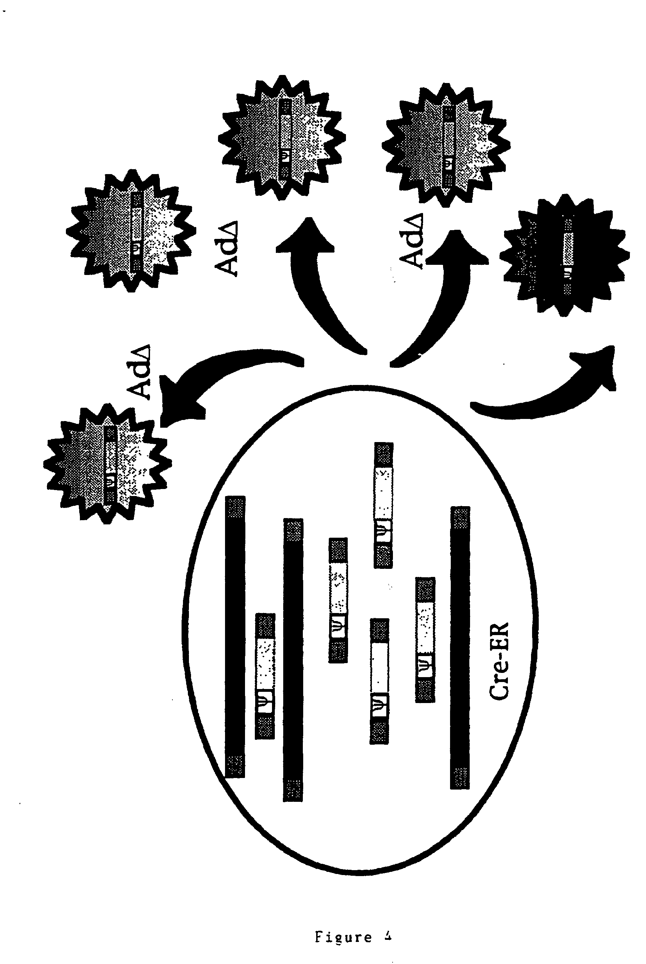

La présente demande décrit en particulier un système de production d'adénovirus minimum utilisant un baculovirus pour délivrer le mini-génome adénoviral à l'aide du système loxP/Cre et un baculovirus pour apporter l'ensemble des fonctions de transcomplémentation (génome complémentant), également à l'aide d'un système Cre/loxP (voir Figures 2-4).

Selon un autre mode de réalisation, le système de recombinaison site-spécifique utilisé pour délivrer les fonctions de complémentation est différent de celui utilisé pour délivrer le génome de l'adénovirus recombinant. En particulier, le système LoxP/Cre peut être utilisé pour délivrer le génome adénoviral défectif et le système AttP/AttB pour délivrer le ou les fonctions de complémentation.

Le procédé de l'invention permet ainsi de construire un vecteur adénoviral délété de toutes séquences virales codantes et ne comportant que les ITRs et le signal d'encapsidation (adénovirus minimum). Ce vecteur peut théoriquement accomoder jusqu'à 37kb de séquence exogène alors que la capacité de clonage des vecteurs actuels ne dépasse pas 8,5kb. Il permet ainsi de cloner des gènes de grande taille comme le gène de la dystrophine (14kb), avec tous leurs éléments de régulation (promoteur, enhancer, introns, ...) afin d'obtenir une expression optimale, dans le tissu cible. De plus l'absence de toute séquence virale immunogène devrait augmenter la durée d'expression du transgène dans les tissus quiescents.

Le génome de l'AAV ou du rétrovirus défectif peut également être introduit sous forme d'un virus, d'un génome, ou d'un plasmide, selon les techniques décrites ci-dessus.

### Cellules compétentes

Le procédé de l'invention peut être mis en oeuvre dans différents types de cellules. On entend au sens de l'invention par "cellule compétente" une cellule permissive à l'infection par le baculovirus et le virus à produire, et permettant un cycle viral productif pour ce dernier. La capacité d'infecter des cellules avec ces virus peut être déterminée en utilisant des virus recombinants exprimant un gène marqueur tel que le gène LacZ de E. coli. Il s'agit de préférence d'une cellule mammifère, encore plus préférentiellement d'une cellule d'origine humaine. Les cellules compétentes utilisées peuvent être des cellules quiescentes ou des cellules en division active, des lignées établies ou des cultures primaires. Il s'agit avantageusement de cellules mammifères compatibles avec une utilisation industrielle, c'est-à-dire sans caractère pathogène reconnu, cultivables et le cas échéant stockables dans des conditions appropriées. Avantageusement, les cellules utilisées sont des cellules hépatiques, musculaires, fibroblastiques, embryonnaires, nerveuses, épithéliales (pulmonaires) ou oculaires (rétiniennes). On peut citer à titre d'exemple non limitatif les cellules 293 ou toute cellule dérivée comportant une fonction de complémentation supplémentaire (293E4, 293E2a, etc), les cellules A549, les cellules HuH7, les cellules Hep3B, les cellules HepG2, les cellules rétinoblastiques humaines (HER, 911), les cellules HeLa, les cellules 3T3 ou encore les cellules KB.

Pour la mise en oeuvre du procédé de l'invention, le génome du virus recombinant et le baculovirus peuvent être introduits dans la population de cellules compétentes de manière simultanée ou espacée dans le temps. Avantageusement, les cellules sont mises en contact à la fois avec le génome recombinant et le baculovirus helper. Dans le cas d'un système générant des molécules réplicatives in vivo, la recombinase est introduite ou exprimée préalablement, simultanément ou ultérieurement.

La production des virus conduit généralement à la lyse des cellules. Les virus produits peuvent donc être récoltés après lyse cellulaire, selon les méthodes connues de purification. Ils peuvent ensuite être conditionnés de différentes manières selon l'usage recherché. Par ailleurs, pour éviter tout risque de contamination du stock viral par des traces éventuelles de baculovirus n'ayant pas pénétré les cellules compétentes (baculovirus helper ou baculovirus apportant le génome viral recombinant), il est possible d'appliquer les techniques suivantes :
- Il est possible de purifier les adénovirus par chromatoghraphie selon la méthode décrite dans la demande FR96/08164. Cette technique permet de séparer l'adénovirus de tout baculovirus résiduel éventuel.
- Il est également possible de faire agir des solvants organiques (par exemple, ether, chloroforme) sur les stocks d'adénovirus purifié. En effet, le baculovirus est un virus enveloppé (enveloppe glycoprotéique), donc très sensible à tout solvant organique (qui extrait les lipides de son enveloppe); au contraire, l'adénovirus n'est pas enveloppé, et les mêmes solvants n'ont aucun effet sur lui.
- Il est encore possible, par purification sur gradient de CsCl, de séparer par densité le baculovirus résiduel éventuel et le virus recombinant.

Ces trois méthodes peuvent être utilisées indépendamment ou conjointement. Par ailleurs, toute autre méthode connue de l'homme du métier peut également être utilisée.

### Utilisation des virus

Les virus ainsi produits peuvent être utilisés pour le clonage, le transfert et l'expression de gènes d'intérêt in vitro, ex vivo ou in vivo. De tels gènes d'intérêt sont par exemple des gènes codant pour des enzymes, des dérivés sanguins, des hormones, des lymphokines : interleukines, interférons, TNF, etc (FR 9203120), des facteurs de croissance, des neurotransmetteurs ou leurs précurseurs ou enzymes de synthèse, des facteurs trophiques : BDNF, CNTF, NGF, IGF, GMF, aFGF, bFGF, NT3, NT5, etc; des apolipoprotéines : ApoAI, ApoAIV, ApoE, etc (WO94/25073), la dystrophine ou une minidystrophine (WO93/06223), des gènes suppresseurs de tumeurs : p53, Rb, Rap1A, DCC, k-rev, etc (WO94/24297), les gènes codant pour des facteurs impliqués dans la coagulation : Facteurs VII, VIII, IX, etc, les gènes suicides : Thymidine kinase, cytosine désaminase, etc; ou encore tout ou partie d'une immunoglobuline naturelle ou artificielle (Fab, ScFv, etc, WO94/29446), etc. Le gène d'intérêt peut également être un gène ou une séquence antisens, dont l'expression dans la cellule cible permet de contrôler l'expression de gènes ou la transcription d'ARNm cellulaires. De telles séquences peuvent par exemple être transcrites, dans la cellule cible, en ARN complémentaires d'ARNm cellulaires et bloquer ainsi leur traduction en protéine, selon la technique décrite dans le brevet EP 140 308. Le gène d'intérêt peut peut aussi être un gène codant pour un peptide antigénique, capable de générer une réponse immunitaire, en vue de la réalisation de vaccins. Il peut s'agir notamment de peptides antigéniques spécifiques du virus d'epstein barr, du virus HIV, du virus de l'hépatite B (EP 185 573), du virus de la pseudo-rage, ou encore spécifiques de tumeurs (EP 259 212). Le gène peut être tout ADN (ADNg, ADNc, etc) codant pour un produit d'intérêt, incluant potentiellement les signaux d'expression appropriés (promoteur, terminateur, etc).

Ces virus peuvent être utilisés in vitro pour la production de ces protéines recombinantes. Ils peuvent également être utilisés, toujours in vitro, pour étudier le mécanisme d'action de ces protéines ou pour étudier la régulation de l'expression de gènes ou l'activité de promoteurs. Ils peuvent également être utilisés in vivo, pour la création de modèles animaux ou d'animaux transgéniques. Ils peuvent également être utilisés pour le transfert et l'expression de gènes in vivo, chez l'animal ou l'homme, dans des approches de thérapie génique ou cellulaire.

La présente demande sera décrite plus en détails à l'aide des exemples qui suivent, qui doivent être considérés comme illustratifs et non limitatifs.

### Légende Des Figures

Figure 1 : Schéma de production d'un adénovirus recombinant défectif de troisième génération (défectif pour les fonctions E1 et E4) utilisant un baculo-E1,E4.
Figures 2-4 : Schéma de production d'un adénovirus minimum utilisant un premier baculovirus pour introduire le génome adénoviral défectif et un second baculovirus pour introduire les fonctions de compléentation.
Figure 5 : Schéma de production d'un AAV recombinant défectif pour les fonctions Rep et Cap utilisant un baculo-Rep/Cap.

### Exemples

### 1. Cellules utilisées

Les cellules utilisées dans le cadre de l'invention peuvent provenir de toute lignée ou population cellulaire infectable par un adénovirus ou un AAV ou un rétrovirus et par un baculovirus, compatible avec une utilisation à des fins thérapeutiques. Il s'agit plus préférentiellement d'une cellule mammifère, notamment humaine. On peut citer plus particulièrement :
- Les cellules de la lignée 293 :
   La lignée 293 est une lignée de cellules embryonnaires de rein humaines contenant l'extrémité gauche (environ 11-12%) du génome de l'adénovirus sérotype 5 (Ad5), comprenant l'ITR gauche, la région d'encapsidation, la région E1, incluant E1a, E1b, la région codant pour la protéine pIX et une partie de la région codant pour la protéine plVa2 (Graham et al., J. Gen. Virol. 36 (1977) 59). Cette lignée est capable de trans-complémenter des adénovirus recombinants défectifs pour la région E1, c'est-à-dire dépourvus de tout ou partie de la région E1, et de produire des stocks viraux ayant des titres élevés
- Les cellules de la lignee A549
   Des cellules complémentant la région E1 de l'adénovirus ont été construites à partir des cellules A549 (Imler et al., Gene Ther. (1996) 75). Ces cellules contiennent un fragment restreint de la région E1, dépourvue de l'ITR gauche, placée sous le controle d'un promoteur inductible.
- Les cellules de la lignée HER
   Le cellules embryonnaires de la rétine humaine (HER) peuvent etre infectées par un adénovirus (Byrd et al., Oncogene 2 (1988) 477). Des cellules d'encapsidation d'adénovirus preparées à partir de ces cellules ont été décrites par exemple dans la demande WO94/28152 ou dans l'article de Fallaux et al. (Htun.Gene Ther. (1996) 215). On peut citer plus particulierement la lignée 911 comprenant la région E1 du genome de l'adénovirus Ad5. du nucléotide 79 au nucléotide 5789 intégrée dans le génome de cellules HER. Cette lignée de cellules permet la production de virus défectifs pour la région E1.
- Les cellules IGRP2
   Les cellules IGRP2 sont des cellules obtenues a partir de cellules 293, par intégration d'une unité fonctionnelle de la région E4 sous contrôle d'un promoteur inductible. Ces cellules permettent la production de virus défectifs pour les régions E1 et E4 (Yeh et al., J. Virol (1996) 70).
- Les cellules VK
   Les cellules VK (VK2-20 et VK10-9) sont des cellules obtenues a partir de cellules 293, par intégration de l'intégralité de la région E4 sous contrôle d'un promoteur inductible, et de la région codant pour la protéine pIX. Ces cellules permettent la production de virus défectifs pour les régions E1 et E4 (Krougliak et al., Hum. Gene Ther. 6 (1995) 1575).
- Les cellules 293E4
   Les cellules 293E4 sont des cellules obtenues a partir de cellules 293, par intégration de l'intégralité de la région E4. Ces cellules permettent la production de virus défectifs pour les régions E1 et E4 (WO95/02697; Cancer Gene Ther. (1995) 322).
- Les cellules Sf9 et Sf21 sont des cellules embryonnaires de lépidoptères. Ces cellules sont accessibles dans des collections (n° CRL-1711 ATCC) ainsi que leurs conditions de culture. Elles sont également disponibles dans le commerce (Gibco). Voir également King et Possee: The baculovirus expression system, London : Chapman et Hall, 1992.
- Les cellules hépatocytaires humaines
   Les cellules HepG2 et Hep3B et HuH7 sont des lignées humaines issues d'hépatocarcinomes. Elles sont accessibles dans les collections de dépot et leurs propriétés ont été décrites par exemple dans les brevets US4,393.133 et US4,393.133.
- Lignée de cellules humaines KB : Issue d'un carcinome épidermique humain, cette lignée est accessible à l'ATCC (ref. CCL17) ainsi que les conditions permettant sa culture.
- Lignée de cellules humaines Hela : Issue d'un carcinome de l'épithelium humain, cette lignée est accessible à l'ATCC (ref. CCL2) ainsi que les conditions permettant sa culture.
- Lignée de cellules W162 : Ces cellules sont des cellules Vero comprenant, intégrée dans leur génome, la région E4 de l'adénovirus Ad2. Ces cellules ont été décrites par Weinberg et al. (PNAS 80 (1983) 5383).

### 2. Infection de cellules humaines par un baculovirus recombinant

Cet exemple décrit la capacité des baculovirus d'infecter des cellules d'origine humaine.

Des cellules humaines (notamment 293 ou ses dérivés) sont infectées avec différentes dilutions d'une solution de baculovirus recombinant exprimant le gène LacZ sous contrôle du LTR du RSV. 48 heures après l'infection, l'apparition de cellules bleues est mise en évidence, démontrant l'infectabilité de ces cellules par un baculovirus.

### 3. Construction de baculovirus exprimant la région E1 de l'adénovirus et d'un adénovirus défectif correspondant.

### 3-1 Clonage de deux cassettes d'expression de E1

Le plasmide AE2 provient du clonage, dans PCRII (Invitrogen), du produit de la PCR faite sur pBRE1 avec les oligonucleotides 5'-TCCTTGCATTTGGGTAACAG-3' et 5'-GCGGCCGCTCAATCTGTATCTTC-3': ce produit de PCR contient les nucléotides 3198 à 3511 d'Ad5, c'est-à-dire l'extrémité 3' de la region E1B. Le plasmide pBRE1 contient les nucléotides 1 à 5788 de l'Ad5 cloné dans pBR322, délété approximativement des nucléotides 1300 à 2300.

Le plasmide AE3 est issu du clonage du fragment NotI-KpnI de AE2. contenant le produit de PCR. dans pCDNA3 (Invitrogen) digéré par NotI-KpnI. Il contient les nucléotides 3198 à 3511 d'Ad5 suivis du site de polyadénylation de la BGH.

Le plasmide AE4 est issu du clonage du fragment BglII-PvuII de AE3 dans pBRE1. AE4 est un plasmide contenant la cassette d'expression de E1 suivante:
- nucléotides : à 3511 de l'Ad5, c'est-à-dire ITR gauche, séquences d'encapsidation, promoteur E1A, gène E1A, promoteur E1B, gène E1B
- le polyA de l'hormone de croissance bovine (BGH) provenant du pCDNA3.

pBRE1 a été digéré par BstNI, puis digéré par la T4 DNA polymérase pour remplir l'extrémité 5' sortante, puis digéré par XbaI. Le fragment ainsi généré contenant les nucléotides 391 à 1339 d'Ad5 a été introduit dans le pic20H digéré par SmaI-XbaI(site non méthylé), pour générer le plasmide AE5.

Le plasmide AE6 est issu du clonage du fragment EcoRI-SmaI de AE5 dans AE4 digéré par EcoRI-SmaI. AE6 est un plasmide contenant la cassette d'expression de E1 suivante:
- nucléotides 391 à 3511 d'Ad5. c'est-à-dire promoteur "réduit" de E1A, gène E1A, promoteur E1B, gène E1B.
- le polyA de l'hormone de croissance bovine (BGH) provenant du pCDNA3.

### 3-2 Clonage de ces deux cassettes E1 dans un baculovirus

Les fragments EcoRI-SphI (l'extrémité 5' sortante SphI ayant été au préalable rendue franche par digestion à la T4 DNA polym'erase) des plasmides AE4 et AE6 sont clonés dans le plasmide pAcSG2 (Pharmingen) entre les sites EcoRI et EcoRV. Ceci génère respectivement les plasmides AE14 et AE15-Dans les deux cas, la région E1 est introduite dans le locus du gène de la polyhédrine (gène+promoteur de la polyhédrine etant délétés).

Les plasmides AE14 et 15 sont cotransfectés avec l'ADN du baculovirus BaculoGold dérivé de la souche AcNPV (Pharmingen) dans des cellules d'insectes Sf9, afin de générer les deux baculovirus recombinants correspondants BacAE14 et BacAE15, porteurs des deux cassettes d'expression de E1 intégrées dans le locus du gène de la polyhédrine.

### 3-3 Clonage d'un Adénovirus recombinant portant une nouvelle délétion E1

Le plasmide pCO1 (WO96/10088) a été digéré par BstXI, puis digéré par la T4 DNA polymérase pour éliminer l'extrémité 3' sortante, puis digéré par RsaI. Le fragment ainsi généré contenant les nucléotides 3513 à 4607 d'Ad5 a été introduit dans pBS-SK+ (Stratagene) linéarisé par EcoRV et digéré par la phosphatase alcaline de veau, pour générer le plasmide AE0.

Le plasmide pMA37 est obtenu par ligation des fragments:
- EcoRV-Nsil de pXL2756, contenant sacB. Le pXL2756, possède le gène de contre-sélection SacB débarrassé de ses sites EcoRI et KpnI, le gène de résistance à la kanamycine, un multisite de clonage et une origine de réplication ColE1.
- NdeI-NsiI de pCO1 (contenant les séquences d'adéno)
- SalI (extrémité 5'-sortante remplie par la T4 DNA polymérase)-Asel de pXL2756 (vecteur kanamycine résistant).

pMA37 contient donc:
- le gène de la résistance à la kanamycine
- le gène SacB conférant une sensibilité au sucrose aux bactéries l'exprimant
- les séquences 1 à 382 (HinfI) d'Ad5 suivies des séquences 3446 à 4415 (NsiI) d'Ad5; il n'y a pas de transgène.

Le plasmide AE1 a été construit par introduction du fragment XhoI-NsiI de AE0 dans le pMA37 digéré par SalI-NsiI. Il contient ainsi:
- le gène de la résistance à la kanamycine
- le gène SacB conférant une sensibilité au sucrose aux bactéries l'exprimant,
- les séquences 1 à 382 (HinfI) d'Ad5 suivies des séquences 3513 à 4415(NsiI) d'Ad5; il n'y a pas de transgène.

A partir du plasmide AE11 sont alors construits les vecteurs navettes suicides (par insertion du transgène d'intérêt) permettant la construction d'adénovirus recombinants par recombinaison dans E. Coli. AE11 ne contient aucune séquence homologue avec le plasmide AE6. Les plasmides AE11 et AE4 ont en commun les séquences 1 à 382 (HinfI) d'Ad5 en amont de E1A, mais il n'y a pas d'homologie en aval de la cassette E1 entre ces deux plasmides. Ainsi, il ne peut y avoir génération de RCA par recombinaison homologue entre un adéno porteur de la délétion E1 existant dans AE11 (c'est-à-dire 382-3513) et les baculovirus BacAE14 ou BacAE15.

### 3-4 Construction d'un Adénovirus recombinant de première génération

Dans un premier temps, un stock de BacAE14 ou 15 est préparé selon les techniques classiques. Ensuite, les cellules compétentes (par exemple HuH7) sont transfectées par 5 µg de plasmide pXL2822 digéré par PacI (le plasmide pXL2822 contient tout l'Ad5 délété pour E1 (382-3446 ou 382-3513) et E3 (28592-30470) et porte une cassette CMV-βGal), et infectées, simultanément ou non, à une MOI comprise entre 10 et 1000, par le BacAE14 ou 15. Lorsque les cellules sont lysées, le surnageant de transfection est récolté, puis appliqué sur des cellules compétentes "fraîches" prélablement ou simultanément infectées avec les BacΔE14 ou 15 (MOI 10 à 1000), afin d'amplifier l'adénovirus Ad2822, et ainsi de suite jusqu'à l'obtention d'un stock d'Ad2822. Le suivi de l'amplification de l'Ad2822 est facilité par la présence de lacZ dans ce virus. A chaque amplification, le surnageant est ainsi pseudo-titré sur W162. Le génome du virus est analysé lors des amplifications afin de vérifier son intégrité. Enfin, cette stratégie présente l'avantage de ne pouvoir générer de RCA dans les stocks d'adénovirus ainsi produits. Cette absence de contamination est également vérifiée.

### 4. Construction d'un baculovirus exprimant les régions E1 et E4 de l'adénovirus

### 4-1 Construction du baculovirus E1,E4 (Bac.E1-E4)

Le protocole utilisé est le suivant : Les régions E1 et E4 sont clonées en orientation inverse au locus du gène de la polyédrine (Ph), dans le vecteur navette pBacPAK8 (Clontech, USA) pour donner pBacE1-E4. Le baculovirus recombiné est ensuite isolé selon les techniques classiques par co-transfection de pBacE1-E4 et de l'ADN du baculovirus BacPAK6 dans les cellules Sf9 (Kitts et Possee, Biotechniques 14(5) (1993) 810). La présence de E1 et E4 dans le génome des baculovirus recombinés est ensuite vérifiée par PCR à partir de cellules Sf9 infectées. La transcription de E1 et E4 dans les cellules Huh7 infectées par le baculovirus recombiné purifié est analysée par RT-PCR à partir des ARN cytoplasmiques.

Pour la construction du baculovirus E1-E4, différents fragments portant E1 peuvent être introduits. Les fragments utilisés dans cet exemple sont ceux décrits dans l'exemple 3 pour la construction du Baculovirus-E1, contenant les régions E1 sous contrôle de leur propre promoteur (promoteur E1a réduit et promoteur E1b). Les fragments E4 utilisés sont les suivants :
- fragment MaeII-MscI 32720-35835 portant l'intégralité de E4
- fragment BglII-PvuII 34115-33126 portant la phase ORF6
- fragment BglII-BgIII 34115-32490 portant les phases ORF6-ORF6/7
- fragment PvuII-AluI 34801-334329 portant la phase ORF3

Ces fragments sont placés alternativement sous le contrôle du promoteur E4 ou de promoteurs différents, notamment du promoteur HSV-TK, CMV ou du LTR-RSV.

Les positions données ci-dessus font référence à la séquence de l'adénovirus Ad5 sauvage telle que publiée et accessible sur base de donnée. Bien que certaines variations mineures puissent exister entre les différents sérotypes d`adénovirus, ces positions sont généralement transposables aux autres sérotypes, et notamment aux Ad2, Ad7, Ad12 et Ad CAV-2.

### 4-2 Transcomplémentation de l'AdΔE1ΔE4-LacZ par le Bac.E1-E4

La production de l'adénovirus AdΔE1ΔE4-LacZ est obtenue par introduction dans les cellules compétentes du baculovirus E1,E4 préparé dans l'exemple 4-1 et du génome adénoviral défectif pour E1 et E4 (voir figure 1). Les conditions optimales de production de l'AdΔE1ΔE4-LacZ dans les cellules compÈtentes, par transcomplémentation par le Bac.E1-E4 purifié sont analysées. Le titre de l'AdΔE1ΔE4 est déterminé en nombre d'unités de transduction de la βgalactosidase (t.d.u.) dans la lignée W162 et l'efficacité de transcomplémentation obtenue est comparée à celle de la lignée d'encapsidation IGRP2.

### 5. Construction d'un baculovirus complémentant l'ensemble du génome de l'adénovirus

Bien que l'expression simultanée de plusieurs protéines, pouvant représenter jusqu'à 13kb de séquence, à partir d'un seul virus ait été rapportée (Belyaev et Roy, NAR 21 (1993) 1219), la capacité maximale de clonage du baculovirus n'est pas très documentée. Cet exemple montre maintenant qu'il est possible de cloner le génome de l'adénovirus délété de son signal d'encapsidation (Ad.Psi-) et bordé par deux sites loxP [loxP-ITR-ITR à E4 -loxp] dans le baculovirus. L'infection de cellules compétentes exprimant la recombinase Cre, de façon inductible ou non (lignée Cre ou Cre-ER, voir exemple 7), par ce baculovirus recombiné et l'activation de la recombinase Cre, permettent l'excision et la circularisation du génome adénoviral. Celui-ci est alors capable de transduire les gènes précoces, de se répliquer et d'activer les gènes tardifs mais incapable d'être encapsidé, et sert ainsi de helper pour la production d'un adénovirus minimum (voir figures 2-4). Dans ce système la production des adénovirus minimum repose sur la co-infection par deux baculovirus et la réalisation de 2 évènements de recombinaison entre les sites loxP. Cette approche présente l'avantage de ne pas générer de particules adénovirales à partir du virus helper.

La construction du génome Ad.Psi- est réalisée chez E. coli. Pour cela, le génome complet de l'adénovirus Ad5 est cloné dans un vecteur de clonage procaryote, ITR jointes. La séquence Psi est délétée par clivage enzymatique et ligation, ou par mutagénèse dirigée. Une séquence LoxP est ensuite introduite de part et d'autre du génome adénoviral, en orientation parallèle. La construction résultante [loxP-ITR-ITR,ΔPsi à E4 -loxP] est ensuite clonée dans un vecteur navette permettant la recombinaison avec un baculovirus, selon la stratégie décrite dans l'exemple 3. Le baculovirus recombinant obtenu, désigné BacAd.Psi-, est ensuite isolé selon les méthodes classiques.

### 6. Construction d'un baculovirus comportant le génome de l'adénovirus recombinant défectif sous forme excisable

Cet exemple décrit la construction d'un baculovirus permettant d'apporter dans les cellules compétentes le génome de l'adénovirus recombinant défectif. Plus particulièrement, l'adénovirus recombinant est défectif pour l'ensemble des régions codantes, et ne retient que les régions ITR et Psi (adénovirus minimum, ou AdΔ).

### 6-1 Construction d'un mini-génome(AdΔ) dans E.Coli

Un plasmide p[IoxP-(ITR-ITR-Psi-P.CMV-Lac2-pA)-IoxP] est construit. Pour cela, une copie de la séquence ITR de l'adénovirus est isolée par coupure enzymatique et/ou amplifiée par PCR, puis clonée en amont de la séquence ITR-Psi contenue dans le vecteur navette de l'adénovirus pGY63. Ce vecteur dérive du pCO1 (WO96/10088) et possède le gène LacZ sous contrôle du promoteur précoce immédiat du cytomégalovirus (P.CMV) terminé par le signal de polyadénylation du virus SV40 (pA), cloné entre la séquence ITR-Psi et le gène codant pour la pIX. La région (ITR-ITR-Psi-P.CMV-LacZ-pA) de ce vecteur (correspondant à un génome d'adénovirus minimum) est ensuite isolée par coupure enzymatique puis clonée entre les sites LoxP dans le multisite de clonage du plasmide pBS246 (Gibco), pour générer le plasmide p[loxP-(ITR-ITR-Psi-P.CMV-LacZ-pA)-loxP]. La capacité de produire des mini-génomes d'adÈnovirus à partir d'un ADN circulaire et de les encapsider est ensuite testée par transfection de ce plasmide dans la lignée IGRP2 infectée par un Ad.ΔE1ΔE4 exprimant la recombinase Cre (AdCre). Les adÈnovirus minimum sont amplifiés par quelques passages successifs du surnageant de la transfection dans la lignée IGRP2. Ils sont ensuite purifiés par centrifugation isopycnique en gradient de chlorure de césium et quantifiés par pseudo-titrage dans la lignée W162. Il est entendu que le gène LacZ peut être aisément substitué par tout autre acide nucléique d'intérêt, par les techniques de biologie moléculaire classiques.

### 6-2 Clonage d'un minigénome excisable dans un baculovirus

La construction portant le mini-génome AdΔ bordée par les deux sites loxP décrite ci-dessus est clonée au locus P10 du baculovirus dans le vecteur navette pAcUW1 (Pharmingen, USA). Le baculovirus Bac.AdΔ est ensuite produit et isolé par les techniques classiques de co-transfection dans les cellules Sf9 précédemment citées, et sélectionné par son phénotype de plage (blanc), après coloration au X-Gal. Ce baculovirus porte donc un génome adénoviral hautement défectif, encadré par deux régions loxP en orientation directe.

### 6-3 Production de l'AdΔ par transcomplémentation avec le baculovirus BacAdPsi-.

Des cellules compétentes sont co-infectées simultanément avec le baculovirus BacAdPsi (décrit dans l'exemple 5), portant les fonctions de transcomplémentation de l'ensemble du génome adénoviral, et avec le baculovirus Bac.AdΔ portant le génome du PseudoAdénovirus (décrit ci-dessus). La recombinase Cre est apportée soit par ajout de la protéine dans le milieu de culture, soit par transfection des cellules avec un plasmide ou un virus (baculovirus) exprimant Cre, soit par expression d'une cassette stablement intégrée dans le génome de la lignée (tel que décrit dans l'exemple 7). L'adénovirus minimum est amplifié par passages successifs des surnageants de culture des cellules co-infectées par BacAd.Psi- et par le surnageant, puis purifié et titré selon les techniques citées précédemment. Cette technique permet d'obtenir, comme seul virus, l'AdΔ, ce qui permet son isolement et sa purification par les méthodes classiques. En outre, les titres obtenus sont compatibles avec une utilisation industrielle.

### 7. Construction d'une lignée exprimant la protéine Cre

Une lignée exprimant Cre, de façon inductible ou non, est construite afin d'augmenter l'efficacité de recombinaison entre les sites loxP dans le baculovirus de l'invention (par exemple le Bac.AdΔ et le BacAd.Psi-) et de contrôler l'expression de Cre. Dans cette construction, Cre est exprimée seule ou sous forme d'une protéine de fusion C-terminale avec le domaine de fixation du récepteur à l'oestradiol (ER), (Metzger et coll., 1996, précitée), sous contrôle d'un promoteur ubiquitaire, de préférence fort, inductible ou non. Plus particulièrement, les promoteurs utilisés sont le promoteur pGRE5, le promoteur de la métallothionine, le promoteur SV40 ou le promoteur du gène HSV-TK.

Pour construire ces lignées Cre, les cellules compÈtentes sont co-transfectées par deux plasmides, l'un contenant la cassette d'expression Cre (Cre ou Cre-ER) et l'autre celle d'un marqueur de sélection (Néo). Des clones résistants au G418 sont sélectionnés, l'activité de Cre dans ces clones est testée par transfection du plasmide p(P.CMV-loxP-ATG-stop-pA-LoxP-LacZ). Ce plasmide contient le gène LacZ inactivé par l'introduction entre le promoteur (P.CMV) et le début de LacZ d'une succession de codons stop dans les trois phases de lecture et du signal de fin de transcription et de polyadénylation du virus SV40, bordés par deux sites loxP. L'expression de Cre dans les clones, en présence ou non de l'inducteur (estradiol), est ensuite révélée par l'activité β-galactosidase induite par la recombinaison entre les deux sites loxP. Plusieurs clones exprimant stablement la protéine de fusion Cre-ER ou la protéine Cre seule à partir des promoteurs et des cellules compétentes précisées ci-après sont ainsi sélectionnés. Ces clones sont utilisables pour la production de virus selon l'invention.

| **Cellule Compétente** | **Promoteur HSV-TK** | | **Promoteur SV40** | | **Promoteur MMTV** | | **Promoteur GRES** | |
|---|---|---|---|---|---|---|---|---|
| **Recombinase** | Cre | Cre-ER | Cre | Cre-ER | Cre | Cre-ER | Cre | Cre-ER |
| 293 | #1 | #7 | #13 | #19 | #25 | #31 | #37 | #43 |
| IGRP2 | #2 | #8 | #14 | #20 | #26 | #32 | #38 | #44 |
| Huf7 | #3 | #9 | #15 | #21 | #27 | #33 | #39 | #45 |
| HepG2 | #4 | #10 | #16 | #22 | #28 | #34 | #40 | #46 |
| HER | #5 | #11 | #17 | #23 | #29 | #35 | #41 | #47 |
| Vero | #6 | #12 | #18 | #24 | #30 | #36 | #42 | #48 |

## Revendications

1. Procédé de production de virus recombinants défectifs selon lequel on introduit, dans une population de cellules compétentes, le génome du virus recombinant défectif et un baculovirus comportant tout ou une partie des fonctions nécessaires à la transcomplémentation du génome recombinant défectif, la partie restante desdites fonctions étant apportée par la cellule compétente.

2. Procédé selon la revendication 1 **caractérisé en ce que** le baculovirus comporte l'ensemble des fonctions nécessaires à la transcomplémentation du génome recombinant défectif.

3. Procédé selon la revendication 1 **caractérisé en ce que** le baculovirus comporte une partie des fonctions nécessaires à la transcomplémentation du génome recombinant défectif, l'autre partie étant apportée par la cellule compétente.

4. Procédé selon la revendication 1 **caractérisé en ce que** les fonctions nécessaires à la transcomplémentation du génome recombinant défectif sont apportées par plusieurs baculovirus.

5. Procédé selon la revendication 1 **caractérisé en ce que** le virus recombinant défectif est un adénovirus recombinant défectif.

6. Procédé selon la revendication 5 **caractérisé en ce que** le génome de l'adénovirus recombinant est défectif pour une ou plusieurs fonctions choisies parmi E1, E2, E3, E4, L1-L5, pIX et IVa2 et le baculovirus comporte l'ensemble des fonctions nécessaires à la transcomplémentation du génome recombinant défectif.

7. Procédé selon la revendication 5 **caractérisé en ce que** le génome de l'adénovirus recombinant est défectif pour une ou plusieurs fonctions choisies parmi E1, E2, E3, E4, L1-L5. pIX et IVa2, le baculovirus comporte une partie des fonctions nécessaires à la transcomplémentation du génome recombinant défectif, l'autre partie des fonctions étant apportées par un ou plusieurs autres baculovirus et/ou par la cellule compétente.

8. Procédé selon la revendication 5 **caractérisé en ce que** le baculovirus helper comporte tout ou une partie de la région E1 de l'adénovirus, permettant la complémentation d'un génome d'adénovirus recombinant défectif pour la région E1.

9. Procédé selon la revendication 5 **caractérisé en ce que** le baculovirus helper comporte tout ou une partie de la région E2 de l'adénovirus, permettant la complémentation d'un génome d'adenovirus recombinant défectif pour la région E2.

10. Procédé selon la revendication 5 **caractérisé en ce que** le baculovirus helper comporte tout ou une partie de la région E4 de l'adénovirus permettant la complémentation d'un génome d'adénovirus recombinant défectif pour la région E4.

11. Procédé selon la revendication 5 **caractérisé en ce que** le baculovirus helper comporte tout ou une partie des régions E1 et E4 de l'adénovirus permettant la complémentation d'un génome d'adénovirus recombinant défectif pour les régions E1 et E4.

12. Procédé selon la revendication 5 **caractérisé en ce que** le génome d'adenovirus recombinant est dépourvu de toute région virale codante et le baculovirus helper comporte l'ensemble des fonctions permettant sa complémentation.

13. Procédé selon la revendication 12 **caractérisé en ce que** le baculovirus comporte l'ensemble d'un génome adènoviral à l'exception de la région d'encapsidation et éventuellement des ITRs.

14. Procédé selon la revendication 1 **caractérisé en ce que** les fonctions de complémentation présentes dans le baculovirus et le génome du virus recombinant défectif ne comportent pas de zone d'homologie susceptible de donner lieu à une recombinaison.

15. Procédé selon la revendication 14 **caractérisé en ce qu'**un génome d'adénovirus recombinant défectif pour la région E1 et éventuellement E3 est introduit dans les cellules compétentes, ces cellules sont infectées, de façon simultanée ou non, par un baculovirus comportant la région E1, la région E1 d'adénovirus présente dans le baculovirus et le génome de l'adénovirus recombinant défectif ne comportant pas de zone d'homologie susceptible de donner lieu à une recombinaison.

16. Baculovirus auxiliaire recombinant tel que défini dans la revendication 1, comprenant inséré dans son génome un acide nucléique codant pour une fonction de complémentation pour la production d'AAVs recombinants défectifs comprenant les régions Rep et Cap de l'AAV sous le contrôle d'un promoteur hétérologue.

17. Baculovirus recombinant tel que défini dans la revendication 1, comprenant inséré dans son génome un acide nucléique codant pour une fonction de complémentation choisie parmi tout ou partie des fonctions codées par les régions gag, pol et env d'un rétrovirus, seules ou en combinaisons, sous le contrôle d'un promoteur hétérologue.

18. Baculovirus selon l'une quelconque des revendications 16 et 17, **caractérisé en ce que** le promoteur est constitué par la région promotrice naturellement responsable de l'expression des fonctions de complémentation.

19. Baculovirus selon l'une quelconque des revendications 16 à 18, **caractérisé en ce que** le promoteur est un promoteur cellulaire ou viral fort, régulé ou non.

20. Baculovirus selon l'une quelconque des revendications 16 à 19, **caractérisé en ce que** l'acide nucléique est introduit au niveau du locus de la polyhédrine ou du locus p10.

21. Baculovirus selon l'une quelconque des revendications 16 à 20, **caractérisé en ce que** l'acide nucléique est introduit sous forme d'une cassette excisable dans la cellule compétente.

22. Baculovirus recombinant selon l'une quelconque des revendications 16 et 17, comprenant insère dans son génome au moins une région d'ADN encadrée par deux séquences permettant une recombinaison site-spécifique et positionnées en orientation directe, ladite région d'ADN comportant au moins une origine de réplication fonctionnelle dans les cellules compétentes et un acide nucléique codant pour une fonction de complémentation d'un virus lesdites séquences permettant une recombinaison site-spécifique sont des séquences LoxP du bactériophage P1, et la recombinaison est obtenue en présence de la protéine Cre.

23. Procédé selon la revendication 5 **caractérisé en ce que** le génome recombinant défectif est introduit dans la cellule par infection avec un adénovirus comportant ledit génome.

24. Procédé selon la revendication 5 **caractérisé en ce que** le génome recombinant défectif est introduit dans la cellule par transfection.

25. Procédé selon la revendication 1 **caractérisé en ce que** le génome recombinant défectif est introduit dans la cellule avec un baculovirus recombinant, distinct du baculovirus portant les fonctions de complémentation.

26. Baculovirus recombinant selon l'une quelconque des revendications 16 à 21 comprenant, inséré dans son génome, au moins une région d'ADN encadrée par deux séquences permettant une recombinaison site-spécifique et positionnées en orientation directe, ladite région d'ADN comportant au moins une origine de réplication fonctionnelle dans les cellules compétentes et un génome d'adénovirus recombinant défectif comprenant essentiellement les régions ITRs, la séquence d'encapsidation et un acide nucléique d'intérêt.

27. Procédé de production d'adénovirus recombinants défectifs **caractérisé en ce que** on infecte une population de cellules compétentes avec un baculovirus selon la revendication 22 et avec un baculovirus selon la revendication 26, on met les cellules on présence de la recombinase permettant la recombinaison site-spécifique, puis on récupère les adénovirus produits.

28. Procédé selon la revendication 1 **caractérisé en ce que** la population de cellules compétentes est une population de cellules hépatiques, musculaires, fibroblastiques, embryonnaires, épithéliales (notamment pulmonaires), oculaires, (notamment rétiniennes) ou nerveuses.

29. Procédé selon la revendication 28 **caractérisé en ce que** la population de cellules compétentes est choisie parmi les cellules 293 ou toute cellule dérivée comportant une fonction de complémentation supplementaire, A549, HuH7, Hep3B, HepG2, HER, 911, HeLa ou KB.

## Patentansprüche

1. Verfahren zur Herstellung von rekombinanten defekten Viren, nach dem man in eine Population kompetenter Zellen das Genom des rekombinanten defekten Virus und ein Baculovirus einführt, das alle Funktionen oder einen Teil der Funktionen enthält, die zur Transkomplementation des rekombinanten defekten Genoms notwendig sind, wobei der restliche Teil der Funktionen durch die kompetente Zelle eingebracht wird.

2. Verfahren nach Anspruch 1, **dadurch gekennzeichnet, dass** das Baculovirus die Gesamtheit der Funktionen enthält, die für die Transkomplementation des rekombinanten defekten Genoms notwendig sind.

3. Verfahren nach Anspruch 1, **dadurch gekennzeichnet, dass** das Baculovirus einen Teil der Funktionen, die für die Transkomplementation des rekombinanten defekten Genoms notwendig sind, enthält, wobei der andere Teil durch die kompetente Zelle eingebracht wird.

4. Verfahren nach Anspruch 1, **dadurch gekennzeichnet, dass** die Funktionen, die zur Transkomplementation des rekombinanten defekten Genoms erforderlich sind, durch mehrere Baculoviren eingebracht werden.

5. Verfahren nach Anspruch 1, **dadurch gekennzeichnet, dass** das rekombinante defekte Virus ein rekombinantes defektes Adenovirus ist.

6. Verfahren nach Anspruch 5, **dadurch gekennzeichnet, dass** das Genom des rekombinanten Adenovirus bezüglich einer Funktion oder mehrerer Funktionen, ausgewählt unter E1, E2, E3, E4, L1-L5, pIX und IVa2, defekt ist und das Baculovirus die Gesamtheit der Funktionen, die für die Transkomplementation des rekombinanten defekten Genoms erforderlich sind, enthält.

7. Verfahren nach Anspruch 5, **dadurch gekennzeichnet, dass** das Genom des rekombinanten Adenovirus bezüglich einer Funktion oder mehrerer Funktionen, ausgewählt unter E1, E2, E3, E4, L1-L5, pIX und IVa2, defekt ist, das Baculovirus einen Teil der Funktionen, die zur Transkomplementation des rekombinanten defekten Genoms erforderlich sind, enthält, wobei der andere Teil der Funktionen durch ein anderes Baculovirus oder mehrere andere Baculoviren und/oder durch die kompetente Zelle eingebracht wird.

8. Verfahren nach Anspruch 5, **dadurch gekennzeichnet, dass** das Helfer-Baculovirus die ganze Region E1 des Adenovirus oder einen Teil der Region E1 des Adenovirus enthält, was die Komplementation eines Genoms von rekombinantem Adenovirus, das bezüglich der Region E1 defekt ist, erlaubt.

9. Verfahren nach Anspruch 5, **dadurch gekennzeichnet, dass** das Helfer-Baculovirus einen Teil der Region E2 des Adenovirus oder die ganze Region E2 des Adenovirus enthält, was die Komplementation eines Genoms von rekombinantem Adenovirus, das bezüglich der Region E2 defekt ist, erlaubt.

10. Verfahren nach Anspruch 5, **dadurch gekennzeichnet, dass** das Helfer-Baculovirus einen Teil der Region E4 des Adenovirus oder die ganze Region E4 des Adenovirus trägt, was die Komplementation eines Genoms von rekombinantem Adenovirus, das bezüglich der Region E4 defekt ist, erlaubt.

11. Verfahren nach Anspruch 5, **dadurch gekennzeichnet, dass** das Helfer-Baculovirus einen Teil der Regionen E1 und E4 des Adenovirus oder die ganzen Regionen E1 und E4 des Adenovirus enthält, was die Komplementation eines Genoms von rekombinantem Adenovirus, das bezüglich der Regionen E1 und E4 defekt ist, erlaubt.

12. Verfahren nach Anspruch 5, **dadurch gekennzeichnet, dass** das Genom von rekombinantem Adenovirus frei von jeder codierenden viralen Region ist und das Helfer-Baculovirus die Gesamtheit der Funktionen enthält, die seine Komplementation erlauben.

13. Verfahren nach Anspruch 12, **dadurch gekennzeichnet, dass** das Baculovirus die Gesamtheit eines Adeno-viralen Genoms mit Ausnahme der Einkapselungsregion und gegebenenfalls der ITRs enthält.

14. Verfahren nach Anspruch 1, **dadurch gekennzeichnet, dass** die Komplementationsfunktionen, die in dem Baculovirus und dem Genom des rekombinanten defekten Virus vorliegen, keine Homologiezone enthalten, die geeignet ist, zu einer Rekombination zu führen.

15. Verfahren nach Anspruch 14, **dadurch gekennzeichnet, dass** ein Genom von rekombinantem Adenovirus, das bezüglich der Region E1 und gegebenenfalls E3 defekt ist, in die kompetenten Zellen eingeführt wird, wobei diese Zellen in gleichzeitiger Art oder nicht durch ein Baculovirus infiziert werden, das die Region E1 enthält, wobei die Adenovirus-Region E1, die im Baculovirus und dem Genom des rekombinanten defektiven Adenovirus vorliegt, keine Homologiezone enthält, die geeignet ist, zu einer Rekombination zu führen.

16. Rekombinantes Helfer-Baculovirus nach Anspruch 1, das in sein Genom eine Nukleinsäure insertiert hat, die für eine Komplementationsfunktion codiert, für die Produktion von rekombinanten defekten AAVs, welche die Regionen Rep und Cap des AAV unter der Kontrolle des heterologen Promotors enthalten.

17. Rekombinantes Baculovirus nach Anspruch 1, das in sein Genom eine Nukleinsäure, die für eine Komplementationsfunktion codiert, ausgewählt unter allen Funktionen oder einem Teil der Funktionen, die durch die Regionen gag, pol und env eines Retrovirus codiert werden, einzeln oder in Kombination, unter der Kontrolle des heterologen Promotors insertiert enthält.

18. Baculovirus nach einem der Ansprüche 16 und 17, **dadurch gekennzeichnet, dass** der Promotor durch die Promotorregion gebildet wird, die natürlicher Weise für die Expression der Komplementationsfunktionen verantwortlich ist.

19. Baculovirus nach einem der Ansprüche 16 bis 18, **dadurch gekennzeichnet, dass** der Promotor ein zellulärer oder stark viraler, regulierter oder nicht-regulierter, Promotor ist.

20. Baculovirus nach einem der Ansprüche 16 bis 19, **dadurch gekennzeichnet, dass** die Nukleinsäure auf der Ebene des Locus des Polyhedrins oder des Locus p10 eingeführt ist.

21. Baculovirus nach einem der Ansprüche 16 bis 20, **dadurch gekennzeichnet, dass** die Nukleinsäure in einer excisierbaren Kassette, in die kompetente Zelle eingeführt wird.

22. Rekombinantes Baculovirus nach einem der Ansprüche 16 und 17, umfassend in sein Genom insertiert wenigstens eine DNA-Region, die von zwei Sequenzen flankiert wird, die eine ortsspezifische Rekombination ermöglichen und in direkter Orientierung positioniert sind, wobei die DNA-Region wenigstens einen Replikationsursprung, der in den kompetenten Zellen funktionell ist, und eine Nukleinsäure, die für eine Komplementationsfunktion eines Virus codiert, enthält, wobei die Sequenzen, die eine ortsspezifische Rekombination ermöglichen, LoxP-Sequenzen des Bacteriophagen P1 sind und die Rekombination in Gegenwart des Proteins Cre erreicht wird.

23. Verfahren nach Anspruch 5, **dadurch gekennzeichnet, dass** das rekombinante defekte Genom durch Infektion mit einem Adenovirus, das das Genom enthält, in die Zelle eingeführt wird.

24. Verfahren nach Anspruch 5, **dadurch gekennzeichnet, dass** das rekombinante defekte Genom durch Transfektion in die Zelle eingeführt wird.

25. Verfahren nach Anspruch 1, **dadurch gekennzeichnet, dass** das rekombinante defekte Genom mit einem rekombinanten Baculovirus, der sich von dem Baculovirus unterscheidet, der die Komplementationsfunktionen trägt, in die Zelle eingeführt wird.

26. Rekombinantes Baculovirus nach einem der Ansprüche 16 bis 21, das in sein Genom wenigstens eine DNA-Region flankiert von zwei Sequenzen, die eine ortsspezifische Rekombination erlauben und in direkter Orientierung positioniert sind, insertiert enthält, wobei die DNA-Region wenigstens einen Replikationsursprung, der in den kompetenten Zellen funktionell ist und ein rekombinantes defektes Adenovirusgenom enthält, welches im Wesentlichen die ITR-Regionen, die Einkapselungssequenz und die Nukleinsäure von Interesse enthält.

27. Verfahren zur Herstellung von rekombinanten defekten Adenoviren, **dadurch gekennzeichnet, dass** man eine Population von kompetenten Zellen mit einem Baculovirus nach Anspruch 22 und mit einem Baculovirus nach Anspruch 26 infiziert, man die Zelle in Gegenwart der Rekombinase bringt, die die ortsspezifische Rekombination ermöglicht, man die produzierten Adenoviren gewinnt.

28. Verfahren nach Anspruch 1, **dadurch gekennzeichnet, dass** die Population der kompetenten Zellen eine Population von hepatischen Zellen, Muskelzellen, Fibrobblasten, Embryonalzellen, Epithelzellen (insbesondere pulmonalen), Augenzellen (insbesondere Bindehautzellen) oder Nervenzellen ist.

29. Verfahren nach Anspruch 28, **dadurch gekennzeichnet, dass** die Population von kompetenten Zellen ausgewählt ist unter den Zellen 293 oder jeder abgeleiteten Zelle, die eine supplementäre Komplementationsfunktion enthält, A549, HuH7, Hep3B, HepG2, HER, 911, HeLa oder KB.

## Claims

1. Process for the production of defective recombinant viruses according to which the genome of the defective recombinant virus and a baculovirus comprising all or some of the functions necessary for the transcomplementation of the defective recombinant genome are introduced into a population of competent cells, the rest of the said functions being provided by the competent cell.

2. Process according to Claim 1, **characterized in that** the baculovirus comprises all the functions necessary for the transcomplementation of the defective recombinant genome.

3. Process according to Claim 1, **characterized in that** the baculovirus comprises some of the functions necessary for the transcomplementation of the defective recombinant genome, the rest being provided by the competent cell.

4. Process according to Claim 1, **characterized in that** the functions necessary for the transcomplementation of the defective recombinant genome are provided by several baculoviruses.

5. Process according to Claim 1, **characterized in that** the defective recombinant virus is a defective recombinant adenovirus.

6. Process according to Claim 5, **characterized in that** the genome of the recombinant adenovirus is defective for one or more functions chosen from E1, E2, E3, E4, L1-L5, pIX and IVa2 and the baculovirus comprises all the functions necessary for the transcomplementation of the defective recombinant genome.

7. Process according to Claim 5, **characterized in that** the genome of the recombinant adenovirus is defective for one or more functions chosen from E1, E2, E3, E4, L1-L5, pIX and IVa2, the baculovirus comprises some of the functions necessary for the transcomplementation of the defective recombinant genome, the rest of the functions being provided by one or more other baculoviruses and/or by the competent cell.

8. Process according to Claim 5, **characterized in that** the helper baculovirus comprises all or part of the E1 region of the adenovirus, allowing the complementation of a recombinant adenovirus genome defective for the E1 region.

9. Process according to Claim 5, **characterized in that** the helper baculovirus comprises all or part of the E2 region of the adenovirus, allowing the complementation of a recombinant adenovirus genome defective for the E2 region.

10. Process according to Claim 5, **characterized in that** the helper baculovirus comprises all or part of the E4 region of the adenovirus, allowing the complementation of a recombinant adenovirus genome defective for the E4 region.

11. Process according to Claim 5, **characterized in that** the helper baculovirus comprises all or part of the E1 and E4 regions of the adenovirus, allowing the complementation of a recombinant adenovirus genome defective for the E1 and E4 regions.

12. Process according to Claim 5, **characterized in that** the recombinant adenovirus genome lacks any coding viral region and the helper baculovirus comprises all the functions allowing its complementation.

13. Process according to Claim 12, **characterized in that** the baculovirus comprises the whole of an adenoviral genome, with the exception of the encapsidation region and possibly of the ITRs.

14. Process according to Claim 1, **characterized in that** the complementation functions present in the baculovirus and the genome of the defective recombinant virus do not comprise a zone of homology capable of giving rise to recombination.

15. Process according to Claim 14, **characterized in that** a recombinant adenovirus genome defective for the E1 and possibly E3 region is introduced into the competent cells, these cells are infected, simultaneously or otherwise, with a baculovirus comprising the E1 region, the adenovirus E1 region present in the baculovirus and the genome of the defective recombinant adenovirus comprising no zone of homology capable of giving rise to recombination.

16. Recombinant helper baculovirus as defined in Claim 1, comprising, inserted into its genome, a nucleic acid encoding a complementation function for the production of defective recombinant AAVs comprising the Rep and Cap regions of the AAV under the control of a heterologous promoter.

17. Recombinant baculovirus as defined in Claim 1, comprising, inserted into its genome, a nucleic acid encoding a complementation function chosen from all or some of the functions encoded by the gag, pol and env regions of a retrovirus, alone or in combinations, under the control of a heterologous promoter.

18. Baculovirus according to either of Claims 16 and 17, **characterized in that** the promoter consists of the promoter region naturally responsible for the expression of the complementation functions.

19. Baculovirus according to any one of Claims 16 to 18, **characterized in that** the promoter is a strong cellular or viral promoter, regulated or otherwise.

20. Baculovirus according to any one of Claims 16 to 19, **characterized in that** the nucleic acid is introduced at the level of the polyhedrin locus or of the p10 locus.

21. Baculovirus according to any one of Claims 16 to 20, **characterized in that** the nucleic acid is introduced in the form of a cassette which is capable of being excised in the competent cell.

22. Recombinant baculovirus according to either of Claims 16 and 17, comprising, inserted into its genome, at least one DNA region flanked by two sequences allowing a site-specific recombination and positioned in direct orientation, the said DNA region comprising at least one replication origin functional in competent cells and a nucleic acid encoding a complementation function of a virus, the said sequences allowing a site-specific recombination are LoxP sequences of the P1 bacteriophage, and the recombination is obtained in the presence of the Cre protein.

23. Process according to Claim 5, **characterized in that** the defective recombinant genome is introduced into the cell by infection with an adenovirus comprising the said genome.

24. Process according to Claim 5, **characterized in that** the defective recombinant genome is introduced into the cell by transfection.

25. Process according to Claim 1, **characterized in that** the defective recombinant genome is introduced into the cell with a recombinant baculovirus, distinct from the baculovirus carrying the complementation functions.

26. Recombinant baculovirus according to any one of Claims 16 to 21, comprising, inserted into its genome, at least one DNA region flanked by two sequences allowing a site-specific recombination and positioned in direct orientation, the said DNA region comprising at least one replication origin functional in competent cells and a defective adenovirus genome comprising essentially the ITR regions, the encapsidation sequence and a nucleic acid of interest.

27. Process for the production of defective recombinant adenoviruses, **characterized in that** a population of competent cells is infected with a baculovirus according to Claim 22 and with a baculovirus according to Claim 26, the cells are put in the presence of the recombinase allowing the site-specific recombination, and then the adenoviruses produced are recovered.

28. Process according to Claim 1, **characterized in that** the population of competent cells is a population of hepatic, muscle, fibroblast, embryonic, epithelial (particularly pulmonary), ocular (particularly retinal) or nerve cells.

29. Process according to Claim 28, **characterized in that** the population of competent cells is chosen from the cells 293 or any derived cell comprising an additional complementation function, A549, HuH7, Hep3B, HepG2, HER, 911, HeLa or KB.
